# EUROPEAN PATENT APPLICATION

(11) **EP 4 438 717 A1**
(43) Date of publication of application: **02.10.2024**
(21) Application number: 22897879.7
(22) Date of filing: 24.11.2022
(51) Int. Cl.: C12N 1/21, C12P 7/46, C12R 1/85

(54) **ACID-RESISTANT YEAST STRAIN FOR HIGH-YIELD PRODUCTION OF SUCCINIC ACID, AND CONSTRUCTION METHOD THEREFOR AND USE THEREOF**

(30) Priority: 24.11.2021 CN 202111404665
(71) Applicant: Tianjin Institute of Industrial Biotechnology, Chinese Academy of Sciences, Tianjin Airport Economic Area Tianjin 300308 (CN)
(72) Inventor: ZHANG, Xueli, Tianjin 300308 (CN); FAN, Feiyu, Tianjin 300308 (CN); XI, Yongyan, Tianjin 300308 (CN); XU, Hongtao, Tianjin 300308 (CN)
(74) Representative: Nederlandsch Octrooibureau
(86) International application number: PCT/CN2022/133973
(87) International publication number: WO 2023/093796

(57) **Abstract**

Provided a genetically modified yeast strain for producing succinic acid, which strain has the activity or an enhanced activity of an NADPH-dependent malate dehydrogenase (EC 1.1.1.82), and optionally also has the activity or an enhanced activity of at least one of the following: (i) soluble fumarate reductase (EC 4.2.1.2), (ii) a pyruvate carboxylase (EC 6.4.1.1), (iii) a fumarase (EC 4.2.1.2), and (iv) succinate transport protein; and a preparation method therefor, a method for producing succinic acid using same, and the use thereof.

## Description

### Technical Field

The present invention relates to the field of biotechnology, and particularly to a novel acid-resistant yeast strain for efficient production of succinate, and a construction method therefor and use thereof.

### Background Art

Succinic acid, known as butanedioic acid, is an intermediate product of the tricarboxylic acid cycle of living organisms, and is also an important organic chemical raw material (Chae T U, Ahn J H, Ko Y S, et al. Metabolic engineering for the production of dicarboxylic acids and diamines [J]. Metabolic Engineering, 2019). Succinic acid is widely used in industries such as food, chemical, agricultural and pharmaceutical industries.

The traditional production method of succinic acid is a chemical method such as paraffin oxidation, hydrogenation of maleic anhydride, carbonylation of acrylic acid, etc, in which a non-renewable petroleum resource is mainly used as a staring material for synthesis. The chemical synthesis method has the disadvantages of high energy consumption, high pollution, etc, and is difficult to achieve the requirement of green manufacturing. In contrast, a biological fermentation method for producing succinic acid has low pollution, low energy consumption and fixed carbon dioxide, which is more in line with a national sustainable development strategy. It is predicted that the global production of succinic acid by biological method can reach 600,000 tons in 2020, with a market value of 539 million US dollars (Hyohak Song, Sang Yup Lee. Production of Succinic Acid by Bacterial Fermentation [J]. Enzyme and Microbial Technology, 2006, 39(3):352-361.).

At present, there are mainly two categories of microbial strains for synthesizing succinic acid: one is fungal/yeast strains, mainly including *Aspergillus niger, Aspergillus fumigatus, Byssochlamys nivea, Pαecilomyces varioti, Saccharomyces cerevisiae,* etc.; and the other is bacteria, including the strains that can naturally produce succinic acid, such as *Actinobacillus succinogenes, Anaerobiospirillum succiniciproducens, Mannheimia succiniciproducens* and *Bacteroides fragilis,* and the strains that can not naturally produce succinic acid, such as *Corynebacterium glutamicum, Escherichia coli ,* etc (Ahn J H, Jang Y S, Lee S Y Production of succinic acid by metabolically engineered microorganisms [J]. Current Opinion in Biotechnology, 2016, 42: 54-66). Filamentous fungi have problems such as slow growth, difficult genetic modification, poor fermentation homogeneity and easy agglomeration of mycelium; the addition of a significant amount of neutralizing agents is requied for the production of succinic acid with bacterial fermentation to maintain the growth of bacterial cells, which increases the cost of isolation and purification and causes a potential pollution problem. Yeast has good acid resistance and can normally grow at pH 3.0, while most of succinic acid is present in the form of free molecules, which can significantly reduce the cost of subsequent isolation and purification.

### Summary of the Invention

In the present invention, the high-efficiency production of succinate is achieved by metabolic engineering modification starting from an acid-resistant yeast *Pichia kudriavzevii* CY902 strain (preserved in China General Microbiological Culture Collection Center (CGMCC) with the deposit number of CGMCC No. 20885) isolated from the epidermis of a wild fruit in Yunnan. Specifically, the modified strain can achieve high-efficiency production of succinate at low pH in a fermentation manner with no or less addition of a neutralizing agent.

In one aspect, the present invention provides a genetically modified succinate-producing yeast strain, having activity or enhanced activity of NADPH-dependent malate dehydrogenase, and optionally further having activity or enhanced activity of at least one of: (i) soluble fumarate reductase activity, (ii) pyruvate carboxylase activity, (iii) fumarase activity, and (iv) succinate transport protein activity.

Preferably, the NDAPH-dependent malate dehydrogenase is derived from a plant, preferably a C4 plant, more preferably a plant of the family Gramineae, Cyperaceae, Compositae, Euphorbiaceae, Chenopodiaceae, Portulacaceae or Amaranthaceae, or is derived from the genus *Euglenα* or *Thermobacillus*, and more preferably is derived from *Sorghum bicolor, Zea mays, Saccharum officinαrum, Pisum sativum, Cicer arietinum, Spinci oleracea, Euglen gracilis,* or *Methanothermobacter thermautotrophicus.*

In one embodiment, the genetically modified succinate-producing yeast strain further has activity-reduced or inactivated pyruvate decarboxylase and/or NAD-dependent glycerol 3-phosphate dehydrogenase.

In a further aspect, the present invention provides a method for constructing a genetically modified succinate-producing yeast strain, comprising imparting an NADPH-dependent malate dehydrogenase activity to the strain or enhancing the NADPH-dependent malate dehydrogenase activity in the strain, and optionally further comprising imparting or enhancing at least one activity of the following: (i) soluble fumarate reductase activity, (ii) pyruvate carboxylase (EC 6.4.1.1) activity, (iii) fumarase (EC 4.2.1.2) activity, and (iv) succinate transport protein activity. Preferably, said NDAPH-dependent malate dehydrogenase is derived from a plant, preferably a C4 plant, more preferably a plant of the family Gramineae, Cyperaceae, Compositae, Euphorbiaceae, Chenopodiaceae, Portulacaceae or Amaranthaceae, or is derived from the genus *Euglenα* or *Thermobacillus,* more preferably *Sorghum bicolor, Zea mays, Saccharum officinarum, Pisum sativum, Cicer arietinum, Spinacia oleracea, Euglena gracilis,* or *Methanothermobacter thermautotrophicus.*

In one embodiment, said method further comprises weakening or inactivating the pyruvate decarboxylase and/or NAD-dependent glycerol 3-phosphate dehydrogenase in the strain.

In a further aspect, the present invention provides a method for producing succinate, comprising culturing the genetically modified succinate-producing yeast strain of the present invention and/or the genetically modified succinate-producing yeast strain obtained by the method for constructing a genetically modified succinate-producing yeast strain according to the present invention (preferably at pH<3.5, for example within a range of pH 1.5-3.5, and/or with no or less addition of a neutralizing agent).

In a further aspect, the present invention provides use of the genetically modified succinate-producing yeast strain according to the present invention and/or the genetically modified succinate-producing yeast strain obtained by the method for constructing a genetically modified succinate-producing yeast strain according to the present invention in the production of succinate (preferably at pH<3.5, for example within a range of pH 1.5-3.5, and/or with or without addition of a neutralizing agent).

### Detailed Description of the Invention

Unless otherwise defined, the technical and scientific terms used herein have the meanings commonly understood by those skilled in the art, for example by referring to Singleton et al., DICTIONARY OF MICROBIOLOGY AND MOLECULAR BIOLOGY 2nd ed., J. Wiley & Sons (New York, NY 1994); Sambrook et al., MOLECULAR CLONING, A LABORATORY MANUAL, Cold Springs Harbor Press (Cold Springs Harbor, NY 1989).

As used herein, the term "genetically modified" means that a strain artificially modified by biological means has one or more modifications, for example gene deletion, amplification or mutation, compared with its initial strain prior to modification, thereby possessing modified biological properties such as improved production property. As used herein, the term "initial strain" can be a native strain to be genetically modified or a strain having other genetic modifications.

As used herein, the term "succinate-producing yeast strain" refers to a yeast strain that can produce succinate under suitable conditions (for example via fermentation) and secrete succinate into an extracellular medium. A succinate-producing yeast strain has a protein that can transport succinate to the outside of the cell, and therefore succinate can be secreted to the outside of the cell after being produced. Suitable succinate transport proteins used for given yeast strains are known in the art, for example including but not limited to a dicarboxylate transport protein SpMAE1 for *Schizosaccharomyces pombe* and dicarboxylate transport protein AnDCT-02 for *Aspergillus niger.*

Succinate-producing yeasts are known in the art, including for example but not limited to the genera *Zygosaccharomyces, Torulopsis, Candida, Pichia, Rhodotroula, Saccharomyces, Yarrowia,* etc. In one embodiment, the succinate-producing yeast strain is a strain from *Pichia, Saccharomyces* or *Yarrowia.* In a preferred embodiment, the succinate-producing yeast strain is *Pichia kudriavzevii* (for example strain CICC32244), *Saccharomyces cerevisiae* (for example strain BY4742) or *Yarrowia lipolytica* (for example strain Polg), for example the *Pichia kudriavzevii* preserved in China General Microbiological Culture Collection Center (CGMCC) with the deposit number of CGMCC No. 20885.

As used herein, "having activity/activites" refers to having detectable activity/activites compared with a reference (for example an initial strain or a wild-type strain) that has no such activity/activites.

As used herein, "having enhanced activity/activities" means that the activity (activities) is increased by at least 5%, at least 10%, at least 20%, at least 30%, at least 40%, at least 50%, at least 60%, at least 70%, at least 80%, at least 90%, at least 100%, at least 150%, at least 200%, at least 250%, at least 300% or more, compared with a reference (for emxaple an initial strain or a wild-type strain) having said activity.

The activity of a protein (for example an enzyme) may be generated or enhanced by any suitable means known in the art, for example including but not limited to expressing or over-expressing a corresponding gene encoding the protein (for example via a vector such as a plasmid) in a strain, introducing a mutation that leads to an increased activity of the protein, etc.

In some embodiments, in the genetically modified succinate-producing yeast strain of the present invention, one or more copies of the target gene or a homologous gene thereof may be integrated into the genome (for example via homologous recombination), optionally at any locus of the genome, (as long as such the integration does not significantly negatively affect the growth and production of the strain), for example where one copy of any gene in the genome is replaced by one or more copies of the target gene or the homologous gene thereof. Those skilled in the art know how to integrate transgenes and select the strains with the transgenes integrated.

As used herein, term "activity-reduced or inactivated ..." or "reduced activity of or inactivated ..." means that the activity is reduced by at least 5%, at least 10%, at least 20%, at least 30%, at least 40%, at least 50%, at least 60%, at least 70%, at least 80%, at least 90%, at least 95%, at least 99% or more, or even 100%, compared with reference activity (for example corresponding activity in an initial strain or a wild-type strain).

The activity of a protein (for example an enzyme) may be reduced or inactivated by any suitable means known in the art, for example including but not limited to using the corresponding weakened or inactivated gene encoding the protein, introducing the mutation that leads to a reduced activity or inactivation of the protein, using an antagonist or inhibitor of the protein (such as an antibody, a ligand, etc), etc.

As used herein, term "weakened or inactivated gene" means that the activity of a gene, for example the expression level (when being used as a protein-encoding gene) or the regulation performance (when being used as a regulator element), is reduced by at least 5%, at least 10%, at least 20%, at least 30%, at least 40%, at least 50%, at least 60%, at least 70%, at least 80%, at least 90%, at least 95%, at least 99% or more, or even is undetectable, compared with a reference (for example a corresponding gene in an initial strain or a wild-type strain). In the case of a gene encoding a protein such as an enzyme, "weakened or inactivated gene" also encompasses that the activity level of the protein expressed by this gene is reduced, for example reduced by at least 5%, at least 10%, at least 20%, at least 30%, at least 40%, at least 50%, at least 60%, at least 70%, at least 80%, at least 90%, at least 95%, at least 99% or more, or even 100%, compared with that of the corresponding protein in an initial strain or a wild-type strain.

Herein, the reference can be a wild-type microorganism or a microorganism prior to performing the desired genetic manipulation (for example an initial microorganism used for performing genetic manipulation to increase gene activity). Herein, parental microorganisms and initial microorganisms can be interchangeably used, and refer to the microorganisms on which the desired genetic manipulation (for example enhancing or weakening the activity of the gene or protein) is performed.

As used herein, malate dehydrogenase (EC 1.1.1.82 (NADPH-dependent)) is encoded by an *MDH* gene, and is invovled in the conversion between oxaloacetate and malate. The NADPH-dependent malate dehydrogenase contributes to the conversion from oxaloacetate to malate, and in this reaction one molecule of NADPH is consumed. The generally used NADPH-dependent malate dehydrogenase sources include C4 plants (for example Gramineae, Cyperaceae, Compositae, Euphorbiaceae, Chenopodiaceae, Portulacaceae and Amaranthaceae plants), *Euglena* and *Thermobacillus,* etc, for example *Sorghum bicolor, Zea mays, Saccharum officinarum, Pisum sativum, Cicer arietinum, Spinacia oleracea, Euglena gracilis* or *Methanothermobacter thermautotrophicus,* etc. As used herein, "having activity or enhanced activity of NADPH-dependent malate dehydrogenase" means that a strain has NDAPH-dependent malate dehydrogenase activity or increased NDAPH-dependent malate dehydrogenase activity that catalyzes the conversion of oxaloacetate into malate.

As used herein, the soluble fumarate reductase (EC 1.3.1.6) is encoded by an *FRD* gene. This enzyme is involved in the interconversion between fumarate and succinate, and contributes to the conversion of fumarate into succinate. Sources of the soluble fumarate reductase that are generally used are yeasts and the order Kinetoplastida, especially *Saccharomyces cerevisiae, Trypanosoma brucei, Leishmania mexicana, Trypanosoma cruzi,* etc. As used herein, "having activity or enhanced activity of fumarate reductase" means that a strain has soluble fumarate reductase activity or increased soluble fumarate reductase activity that converts fumarate into succinate.

As used herein, the fumarase (EC 4.2.1.2) is encoded by an *FUM* gene. This enzyme is involved in the interconversion between fumarate and malate in the cytoplasm and mitochondria, and contributes to the conversion of malate into fumarate. The generally used sources are *Actinobacillus succinogenes, Mannheimia succiniciproducens, Escherichia coli, Pichia kudriavzevii, Rhizopus oryzae,* etc. It is known that one FUM gene *FUM1* is present in *Pichia kudriavzevii.* As used herein, "having activity or enhanced activity of fumarase" means that a strain has fumarase activity or increased fumarase activity that converts malate into fumarate.

As used herein, the "succinate transport protein" refers to a protein capable of transporting intracellular succinate to the outside of the cell, including for example but not limited to SpMAE1 protein of *Schizosaccharomyces pombe* (Uniprot database search number: P50537), dicarboxylate transport protein AnDCT-02 of *Aspergillus niger* (NCBI Reference Sequence: XP_001398131.1) and dicarboxylate transport proteins EcDcuB (Gene ID: 948641) and EcDcuC (Gene ID: 945000) of *Escherichia coli,* etc. Herein, "having avtivity or enhanced avtivity of succinate transport protein" means that a strain has an activity or an increased activity that transports succinate to the outside of the cell.

As used herein, the dicarboxylate transport protein SpMAE1 protein of *Schizosaccharomyces pombe* is encoded by an *SpMAE1* gene, and the SpMAE1 protein contributes to transporting the intracellular dicarboxylate to the outside of the cell. Herein, "having activity or enhanced activity of SpMAE1" means that a strain has activity or increased activity that transports dicarboxylate to the outside of the cell.

As used herein, the pyruvate carboxylase (EC 6.4.1.1) is encoded by a *PYC* gene. The pyruvate carboxylase is invoved in the interconversion between oxaloacetate and pyruvate in the process of gluconeogenesis, and contributes to the conversion of pyruvate and carbon dioxide into oxaloacetate. Sources of the pyruvate carboxylase that are generally used include fungi, especially yeasts and filamentous fungi, preferably *Saccharomyces cerevisiae, Pichia kudriavzevii, Aspergillus oryzae, Kluyveromyces marxianus,* etc. It is known that one PYC gene, i.e., *PYC1* gene, is present in *Pichia kudriavzevii.* Herein, "having activity or enhanced activity of pyruvate carboxylase" means that a strain has activity or increased activity that converts pyruvate into oxaloacetate.

As used herein, pyruvate decarboxylase (EC 4.1.1.43) is encoded by a *PDC* gene, and is involved in the decarboxylation of pyruvate in the pathway of ethanol synthesis. It is known that one *PDC* gene present in *Pichia kudriavzevii* is *PDC1* gene. Herein, the "activity-reduced or inactivated pyruvate decarboxylase" refers to a reduction or loss of pyruvate decarboxylation activity of this enzyme.

As used herein, NAD-dependent glycerol 3-phosphate dehydrogenase (EC 1.1.1.8) is encoded by a *GPD* gene, and is involved in the interconversion between glycerone phosphate and glycerol 3-phosphate in the pathway of glycerol synthesis. It is known that one *GPD* gene, i.e., *GPD1* gene, is present in *Pichia kudriavzevii.* Herein, the "activity-reduced or inactivated NAD-dependent glycerol 3-phosphate dehydrogenase" refers to a reduction or loss of the activity of this enzyme that catalyzes the interconversion between glycerone phosphate and glycerol 3-phosphate.

As used herein, the orotidine 5'-phosphate decarboxylase (EC 4.1.1.23) is encoded by a *URA3* gene, and is involved in decarboxylation reaction of orotidine 5'-phosphate in the process of pyrimidine synthesis. It is known that one *URA3* gene is present in *Pichia kudriavzevii.* Herein, the "activity-reduced or inactivated orotidine 5'-phosphate decarboxylase" refers to a reduction or loss of the activity of this enzyme that catalyzes orotidine 5'-phosphate decarboxylation reaction.

As used herein, the alcohol dehydrogenase 1 (EC 1.1.1.1) is encoded by an *ADH1* gene, and is involved in the interconversion between acetaldehyde and ethanol in the pathway of ethanol synthesis. Herein, the "activity-reduced or inactivated alcohol dehydrogenase 1" refers to a reduction or loss of the activity of this enzyme that catalyzes interconversion between acetaldehyde and ethanol. In one embodiment, the *ADH1* gene in the strain is knocked out, for example by homologous recombination.

As used herein, the monocarboxylate permease (NCBI Reference Sequence XP_029320775.1) is encoded by a *MCH4* gene. Herein, the "activity-reduced or inactivated monocarboxylate permease" refers to a reduction or loss of the catalytic activity of this enzyme. In one embodiment, the *MCH4* gene in the strain is knocked out, for example by homologous recombination.

As used herein, a *JEN2* gene encodes dicarboxylate transport protein, which is involved in the process of transporting dicarboxylate from the culture medium into the cells. It is known that there are two *JEN2* genes (*JEN2-1* (encoding a polypetide of SEQ ID NO: 14) and *JEN2-2* (encoding a polypetide of SEQ ID NO: 15)) in *Pichia kudriavzevii.* Herein, the "activity-reduced or inactivated dicarboxylate transport protein" refers to a reduction or loss of the activity of a cell that transport dicarboxylate from the culture medium into the cell.

As used herein, the "neutralizing agent" refers to a reagent that precipitates succinate in the form of calcium succinate from a fermentation system. It is known in the art that a substance that can be used as the neutralizing agent includes but is not limited to calcium carbonate.

As used herein, "no or less addition of a neutralizing agent" refers to an amount of a neutralizing agent that is at least 5%, at least 10%, at least 20%, at least 30%, at least 40%, at least 50%, at least 60%, at least 70%, at least 80%, at least 90%, at least 95%, at least 99% or more, or even 100% lower than the amount of the neutralizing agent known in the art that is added for the fermentation production of succinate. For example, the amount of a neutralizing agent added in the method of the present invention can be 0-30 g/L.

As used herein, terms "polypeptide", "amino acid sequence", "peptide" and "protein" can be interchangeablely used herein, and refer to an amino acid chain with any length which may contain a modified amino acid and/or may be interrupted by a non-amino acid. This term also encompasses an amino acid chain that has been modified by naturally or artificially intervention; for example, disulfide bond formation, glycosylation, lipidation, acetylation, phosphorylation, or any other manipulations or modifications, such as conjugation with a marker component.

As used herein, the expressions "gene", "nucleic acid sequence", "polynucleotide" and "nucleotide sequence" can be interchangeablely used herein, and refer to a nucleotide chain including DNA and RNA. "Expression of gene" or "gene expression" refers to the transcription of a DNA region operatively linked to a suitable regulatory region, especially a promoter, into biological activitive RNA and RNA that can be translated into a biological activitive protein or peptide.

As used herein, the "degenerate sequence" refers to a nucleotide sequence that encodes the same amino acid sequence as a specified sequence but has a different nucleotide sequence due to the degeneracy of genetic codons.

As used herein, the terms "homology", "sequence identity" and the like are interchangeablely used herein. The sequence identity may be detected by alignment of the numbers of the identical nucleotide bases between a polynucleotide and a reference polynucleotide, for example as determined by a standard alignment algorithm program using default gap penalties established by each supplier. Whether or not two nucleic acid molecules have at least 80%, at least 85%, at least 90%, at least 95%, at least 96%, at least 97%, at least 98%, or at least 99%, "identical" nucleotide sequence can be determined by using a known computerized algorithm such as BLASTN, FASTA, DNAStar and Gap (University of Wisconsin Genetics Computer Group (UWG), Madison WI, USA). For example, the identity percentage of a nucleic acid molecule can be determined, for example, by comparing sequence information using GAP computer program (e.g., Needleman et al. J. Mol. Biol. 48: 443 (1970), revised by Smith and Waterman (Adv. Appl. Math. 2: 482 (1981)). In brief, the GAP program defines similarity depending on the number of aligned similar symbols (i.e., nucleotides) divided by the total number of symbols in the shorter sequence of the two sequences.

As used herein, *Pk2365* gene encodes a bifunctional enzyme of oxaloacetate decarboxylase and 3-hydroxy-3-methylglutarate aldolase (EC 4.1.3.17 or 4.1.1.112). Herein, the "activity-reduced or inactivated bifunctional enzyme of oxaloacetate decarboxylase and 3-hydroxy-3-methylglutarate aldolase" refers to a reduction or loss of activity of this enzyme that catalyzes oxaloacetate decarboxylation and aldol condensation of 3-hydroxy-3-methylglutarate.

In one aspect, the present invention provides a genetically modified succinate-producing yeast strain, having activity or enhanced activity of NADPH-dependent malate dehydrogenase, and optionally further having activity or enhanced activity of at least one of: (i) soluble fumarate reductase activity, (ii) pyruvate carboxylase activity, (iii) fumarase activity, and (iv) succinate transport protein activity. The term "at least one" includes any one, two, three or all four activities selected therefrom.

In one embodiment, the present invention provides a genetically modified succinate-producing yeast strain, having activities or enhanced activities of NADPH-dependent malate dehydrogenase and soluble fumarate reductase.

In one embodiment, the present invention provides a genetically modified succinate-producing yeast strain, having activities or enhanced activities of NADPH-dependent malate dehydrogenase and pyruvate carboxylase.

In one embodiment, the present invention provides a genetically modified succinate-producing yeast strain, having activities or enhanced activities of NADPH-dependent malate dehydrogenase and fumarase.

In one embodiment, the present invention provides a genetically modified succinate-producing yeast strain, having activities or enhanced activities of NADPH-dependent malate dehydrogenase and succinate transport protein.

In one embodiment, the present invention provides a genetically modified succinate-producing yeast strain, having activities or enhanced activities of NADPH-dependent malate dehydrogenase, soluble fumarate reductase and pyruvate carboxylase.

In one embodiment, the present invention provides a genetically modified succinate-producing yeast strain, having activities or enhanced activities of NADPH-dependent malate dehydrogenase, soluble fumarate reductase and fumarase.

In one embodiment, the present invention provides a genetically modified succinate-producing yeast strain, having activities or enhanced activities of NADPH-dependent malate dehydrogenase, soluble fumarate reductase and succinate transport protein.

In one embodiment, the present invention provides a genetically modified succinate-producing yeast strain, having activities or enhanced activities of NADPH-dependent malate dehydrogenase, pyruvate carboxylase and fumarase.

In one embodiment, the present invention provides a genetically modified succinate-producing yeast strain, having activities or enhanced activities of NADPH-dependent malate dehydrogenase, pyruvate carboxylase and succinate transport protein.

In one embodiment, the present invention provides a genetically modified succinate-producing yeast strain, having activities or enhanced activities of NADPH-dependent malate dehydrogenase, fumarase and succinate transport protein.

In one embodiment, the present invention provides a genetically modified succinate-producing yeast strain, having activities or enhanced activities of NADPH-dependent malate dehydrogenase, soluble fumarate reductase, pyruvate carboxylase and fumarase.

In one embodiment, the present invention provides a genetically modified succinate-producing yeast strain, having activities or enhanced activities of NADPH-dependent malate dehydrogenase, soluble fumarate reductase, pyruvate carboxylase and succinate transport protein.

In one embodiment, the present invention provides a genetically modified succinate-producing yeast strain, having activities or enhanced activities of NADPH-dependent malate dehydrogenase, soluble fumarate reductase, fumarase, and succinate transport protein.

In one embodiment, the present invention provides a genetically modified succinate-producing yeast strain, having activities or enhanced activities of NADPH-dependent malate dehydrogenase, pyruvate carboxylase, fumarase, and succinate transport protein.

In one embodiment, the present invention provides a genetically modified succinate-producing yeast strain, having activities or enhanced activities of NADPH-dependent malate dehydrogenase, soluble fumarate reductase, pyruvate carboxylase, fumarase, and succinate transport protein.

In one embodiment, having activity (activites) or enahnced activity (activites) is achieved by expressing or over-expressing the corresponding encoding gene in the strain. Thus, in one embodiment, a gene encoding NADPH-dependent malate dehydrogenase is expressed or over-expressed in the genetically modified succinate-producing yeast strain.

In one embodiment, the NADPH-dependent malate dehydrogenase is derived from plants (preferably C4 plants, more preferably Gramineae, Cyperaceae, Compositae, Euphorbiaceae, Chenopodiaceae, Portulacaceae and Amaranthaceae plants), *Euglenα* or *Thermobacillus,* preferably is derived from *Sorghum bicolor, Zea mays, Saccharum officinarum, Pisum sativum, Cicer arietinum, Spinαciα oleracea, Euglenα gracilis or Methanothermobacter thermautotrophicus,* more preferably is derived from *Sorghum bicolor.*

In one embodiment, the activity of NADPH-dependent malate dehydrogenase is generated or increased by expressing or over-expressing an *MDH* gene encoding NADPH-dependent malate dehydrogenase in the genetically modified succinate-producing yeast strain. Preferably, the *MDH* gene encoding the NADPH-dependent malate dehydrogenase is preferably derived from plants, preferably C4 plants, more preferably Gramineae, Cyperaceae, Compositae, Euphorbiaceae, Chenopodiaceae, Portulacaceae or Amaranthaceae plants, or is derived from *Euglenα* or *Thermobacillus,* and more preferably is derived from *Sorghum bicolor, Zea mays, Saccharum officinαrum, Pisum sativum, Cicer αrietinum, Spinαciα oleracea, Euglena gracilis* or *Methanothermobacter thermautotrophicus,* and more preferably is derived from *Sorghum bicolor.*

In one embodiment, said *MDH* gene encoding NADPH-dependent malate dehydrogenase comprises the sequence of SEQ ID NO: 1 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having NADPH-dependent malate dehydrogenase activity.

In one embodiment, said *MDH* gene encoding NADPH-dependent malate dehydrogenase is incorporated into the genome of the genetically modified succinate-producing yeast (for example *Pichia kudriαvzevii*)*,* for example at a position of *Pk2365* loci. Said *MDH* gene encoding NADPH-dependent malate dehydrogenase can be placed under the control of a suitable promoter (for example the promoter of *FBA1* gene (as set forth in SEQ ID NO: 17)) and/or a terminator (for example the terminator of *INO1* gene (as set forth in SEQ ID NO: 18)).

In a further embodiment, the genetically modified succinate-producing yeast strain further has soluble fumarate reductase or has enhanced soluble fumarate reductase. In one embodiment, the soluble fumarate reductase is derived from yeasts and the order Kinetoplastida, for example but is not limited to *Saccharomyces cerevisiae, Trypanosoma brucei, Leishmania mexicana* and *Trypanosoma cruz*i*.*

In one embodiment, the soluble fumarate reductase activity is generated or increased by expressing or over-expressing a gene encoding soluble fumarate reductase in the genetically modified succinate-producing yeast strain. Preferably, said gene encoding soluble fumarate reductase is preferably derived from yeasts and the order Kinetoplastida, for example but is not limited to *Saccharomyces cerevisiae, Trypanosoma brucei, Leishmania mexicana* and *Trypanosoma cruzi.*

In one embodiment, a glyoxysome-localized peptide at the 3' end of the soluble fumarate reductase is partially or completely truncated, so that it is freely present in the cytoplasm.

In one embodiment, the gene encoding soluble fumarate reductase comprises a sequence of any one of SEQ ID Nos: 3-5 and a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having soluble fumarate reductase activity.

In one embodiment, the gene encoding soluble fumarate reductase is incorporated into the genome of the genetically modified succinate-producing yeast (for example *Pichia kudriavzevii*), for example at a position of *ADH1* loci. The gene encoding soluble fumarate reductase can be placed under the control of a suitable promoter (for example the promoter of *ADH1* gene (as set forth in SEQ ID NO: 21)) and/or a terminator (for example the terminator of *ADH1* gene (as set forth in SEQ ID NO: 22)).

In a further embodiment, the genetically modified succinate-producing yeast strain further has succinate transport protein activity or enhanced succinate transport protein activity.

In one embodiment, the succinate transport protein is selected from a group consisting of a SpMAE1 protein, an AnDCT-02 protein, an EcDcuB protein and an EcDcuC protein. In one embodiment, the succinate transport protein activity is generated or increased by expressing or over-expressing a gene encoding succinate transport protein such as the SpMAE1 protein, in the genetically modified succinate-producing yeast strain. In one embodiment, the succinate transport protein activity is generated or enhanced by expressing or over-expressing *SpMAE1* gene.

In one embodiment, said *SpMAE1* gene comprises the sequence of SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid having succinate transport protein activity (for example derived from *Schizosaccharomyces pombe*)*.* Optionally, said *SpMAE1* gene is incorporated into the genome of the genetically modified succinate-producing yeast strain (for example *Pichia kudriavzevii*), for example at a position of *MCH4* loci. The *SpMAE1* gene can be placed under the control of a suitable promoter (for example the promoter of *TDH3* gene (as set forth in SEQ ID NO: 19)) and/or a terminator (for example the terminator of *GAL2* gene (as set forth in SEQ ID NO: 20)).

In a further embodiment, the genetically modified succinate-producing yeast strain further has pyruvate carboxylase activity or has enhanced pyruvate carboxylase activity. Said pyruvate carboxylase activity can be generated or enhanced by expressing or over-expressing a gene encoding pyruvate carboxylase. The pyruvate carboxylase can be derived from fungi, especially yeasts and filamentous fungi, preferably *Saccharomyces cerevisiae, Pichia kudriavzevii, Aspergillus oryzae, Kluyveromyces marxianus,* etc. In one embodiment, the gene encoding pyruvate carboxylase can be selected from a group consisting of the *PYC* gene of *Aspergillus oryzae* and the *PYC1* gene of *Pichia kudriavzevii.*

In one embodiment, the pyruvate carboxylase comprises an amino acid sequence encoded by the sequence of SEQ ID NO: 6 or 7, or an amino acid sequence having at least 75%, 80%, 85%, 90%, 91%, 92%, 93%, 94%, 95%, 96%, 97%, 98%, 99% or more identity thereto and having pyruvate carboxylase activity.

In one embodiment, the gene encoding pyruvate carboxylase comprises the sequence of SEQ ID NO: 6 or 7 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid having pyruvate carboxylase activity.

Optionally, the gene encoding pyruvate carboxylase is incorporated into the genome of the genetically modified succinate-producing yeast (for example *Pichia kudriavzevii*), for example at a position of *JEN2-1* loci. The gene encoding pyruvate carboxylase can be placed under the control of a suitable promoter (for example the promoter of *TDH3* gene (as set forth in SEQ ID NO: 19)) and/or a terminator (for example the terminator of *GAL2* gene (as set forth in SEQ ID NO: 20)).

In a further embodiment, the genetically modified succinate-producing yeast strain further has fumarase activity or has enhanced fumarase activity. The fumarase activity can be generated or enhanced by expressing or over-expressing a gene encoding fumarase. In one embodiment, the genetically modified succinate-producing yeast strain expresses or over-expresses the gene encoding fumarase. In one embodiment, the fumarase is derived from for example but not limited to *Actinobacillus succinogenes, Mannheimia succiniciproducens, Escherichia coli, Pichia kudriavzevii* and *Rhizopus oryzae.*

In one embodiment, the fumarase comprises the amino acid sequence of SEQ ID NO: 95, or an amino acid sequence having at least 75%, 80%, 85%, 90%, 91%, 92%, 93%, 94%, 95%, 96%, 97%, 98%, 99% or more identity thereto and having fumarase activity.

In one embodiment, the mitochondrion-localized peptide at the 5' end of said fumarase is partially or completely truncated, so that it cannot localize to the mitochondrion but is freely present in the cytoplasm.

In one embodiment, the gene encoding fumarase comprises the sequence of SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having fumarase activity.

Optionally, said gene encoding fumarase is incorporated into the genome of the genetically modified succinate-producing yeast (for example *Pichia kudriαvzevii*), for example at a position of *PDC1* loci. The gene encoding fumarase can be placed under the control of a suitable promoter (for example the promoter of *TDH3* gene (as set forth in SEQ ID NO: 19)) and/or a terminator (for example the terminator of *GAL2* gene (as set forth in SEQ ID NO: 20)).

The gene that is required to be expressed or over-expressed can be integrated at a suitable position in the strain genome, as long as such integration does not negatively affect the growth, proliferation and/or productivity of the strain. For example, it can be integrated at any one or more positions of the genomes encoding the following proteins: (i) pyruvate decarboxylase (EC 4.1.1.43), (ii) NAD-dependent glycerol 3-phosphate dehydrogenase (EC 1.1.1.8), (iii) orotidine 5'-phosphate decarboxylase (EC 4.1.1.23), (iv) monocarboxylate permease, (v) dicarboxylate transport protein, (vi) alcohol dehydrogenase 1 (EC 1.1.1.1), and (vii) bifunctional enzyme of oxaloacetate decarboxylase and 3-hydroxy-3-methylglutarate aldolase (EC 4.1.3.17 or 4.1.1.112).

In a further embodiment, the genetically modified succinate-producing yeast strain further has at least one reduced activity of or inactivated: (i) pyruvate decarboxylase (EC 4.1.1.43), (ii) NAD-dependent glycerol 3-phosphate dehydrogenase (EC 1.1.1.8), (iii) orotidine 5'-phosphate decarboxylase (EC 4.1.1.23), (iv) monocarboxylate permease, (v) dicarboxylate transport protein, (vi) alcohol dehydrogenase 1 (EC 1.1.1.1), and (vii) bifunctional enzyme of oxaloacetate decarboxylase and 3-hydroxy-3-methylglutarate aldolase (EC 4.1.3.17 or 4.1.1.112).

In one embodiment, the genetically modified succinate-producing yeast strain has activity-reduced or inactivated pyruvate decarboxylase. The activity-reduced or inactivated pyruvate decarboxylase can be achieved by weakening or inactivating the gene encoding pyruvate decarboxylase in the strain. Thus, in one embodiment, the genetically modified succinate-producing yeast strain has a weakened or inactivated gene encoding pyruvate decarboxylase. In one embodiment, the gene encoding pyruvate decarboxylase for example *PDC1* gene in the genetically modified succinate-producing yeast strain, is knocked out.

In one embodiment, the pyruvate decarboxylase comprises an amino acid sequence of SEQ ID NO: 10, or an amino acid sequence having at least 75%, 80%, 85%, 90%, 91%, 92%, 93%, 94%, 95%, 96%, 97%, 98%, 99% or more identity thereto and having pyruvate decarboxylase activity.

In one embodiment, the gene encoding pyruvate decarboxylase encodes a protein of SEQ ID NO: 10, or an amino acid sequence having at least 75%, 80%, 85%, 90%, 91%, 92%, 93%, 94%, 95%, 96%, 97%, 98%, 99% or more identity thereto and having pyruvate decarboxylase activity.

In one embodiment, the genetically modified succinate-producing yeast strain further has activity-reduced or inactivated NAD-dependent glycerol 3-phosphate dehydrogenase. In one embodiment, the genetically modified succinate-producing yeast strain has a weakened or inactivated gene encoding NAD-dependent glycerol 3-phosphate dehydrogenase. In one embodiment, the gene encoding NAD-dependent glycerol 3-phosphate dehydrogenase in the genetically modified succinate-producing yeast strain (for example *Pichia kudriavzevii*) is knocked out.

In one embodiment, the NAD-dependent glycerol 3-phosphate dehydrogenase comprises an amino acid sequence of SEQ ID NO: 11, or an amino acid sequence having at least 75%, 80%, 85%, 90%, 91%, 92%, 93%, 94%, 95%, 96%, 97%, 98%, 99% or more identity thereto and having NAD-dependent glycerol 3-phosphate dehydrogenase activity.

In one embodiment, the gene encoding the NAD-dependent glycerol 3-phosphate dehydrogenase encodes for example a protein of SEQ ID NO: 10, or an amino acid sequence having at least 75%, 80%, 85%, 90%, 91%, 92%, 93%, 94%, 95%, 96%, 97%, 98%, 99% or more identity thereto and having NAD-dependent glycerol 3-phosphate dehydrogenase activity.

In a further embodiment, the genetically modified succinate-producing yeast strain further has activity-reduced or inactivated alcohol dehydrogenase 1. In one embodiment, the genetically modified succinate-producing yeast strain has a weakened or inactivated gene encoding alcohol dehydrogenase 1. In one embodiment, the gene encoding alcohol dehydrogenase 1 in the genetically modified succinate-producing yeast strain (for example *Pichia kudriavzevii*) is knocked out.

In one embodiment, the alcohol dehydrogenase 1 comprises an amino acid sequence of SEQ ID NO: 12, or an amino acid sequence having at least 75%, 80%, 85%, 90%, 91%, 92%, 93%, 94%, 95%, 96%, 97%, 98%, 99% or more identity thereto and having alcohol dehydrogenase 1 activity.

In one embodiment, the gene encoding alcohol dehydrogenase 1 encodes for example a protein of SEQ ID NO: 12, or an amino acid sequence having at least 75%, 80%, 85%, 90%, 91%, 92%, 93%, 94%, 95%, 96%, 97%, 98%, 99% or more identity thereto and having alcohol dehydrogenase 1 activity.

In a further embodiment, the genetically modified succinate-producing yeast strain further has orotidine 5'-phosphate decarboxylase (EC 4.1.1.23), dicarboxylate transport protein, bifunctional enzyme of oxaloacetate decarboxylase and 3-hydroxy-3-methylglutarate aldolase, and/or monocarboxylate permease, which are activity-reduced or inactivated.

In a further embodiment, the gene encoding orotidine 5'-phosphate decarboxylase and/or the gene encoding dicarboxylate transport protein (for example a *JEN2* gene such as *JEN2-1* gene, *JEN2-2* gene) and/or the gene encoding monocarboxylate permease and/or the gene encoding bifunctional enzyme of oxaloacetate decarboxylase and 3-hydroxy-3-methylglutarate aldolase in the genetically modified succinate-producing yeast strain is knocked out.

In one embodiment, the genetically modified succinate-producing yeast strain further has activity-reduced or inactivated bifunctional enzyme of oxaloacetate decarboxylase and 3-hydroxy-3-methylglutarate aldolase. Specifically, the genetically modified succinate-producing yeast strain has a weakened or inactivated gene encoding bifunctional enzyme of oxaloacetate decarboxylase and 3-hydroxy-3-methylglutarate aldolase. Preferably, the gene encoding the bifunctional enzyme of oxaloacetate decarboxylase and 3-hydroxy-3-methylglutarate aldolase in the genetically modified succinate-producing yeast strain (for example *Pichia kudriavzevii*) is knocked out.

In one embodiment, the bifunctional enzyme of oxaloacetate decarboxylase and 3-hydroxy-3-methylglutarate aldolase comprises the amino acid sequence of SEQ ID NO: 9, or an amino acid sequence having at least 75%, 80%, 85%, 90%, 91%, 92%, 93%, 94%, 95%, 96%, 97%, 98%, 99% or more identity thereto and having the activity of bifunctional enzyme of oxaloacetate decarboxylase and 3-hydroxy-3-methylglutarate aldolase.

In one embodiment, the gene encoding bifunctional enzyme of oxaloacetate decarboxylase and 3-hydroxy-3-methylglutarate aldolase encodes a protein of SEQ ID NO: 9, or an amino acid sequence having at least 75%, 80%, 85%, 90%, 91%, 92%, 93%, 94%, 95%, 96%, 97%, 98%, 99% or more identity thereto and having the activity of bifunctional enzyme of oxaloacetate decarboxylase and 3-hydroxy-3-methylglutarate aldolase.

In one embodiment, the genetically modified succinate-producing yeast strain further has activity-reduced or inactivated orotidine 5'-phosphate decarboxylase. Specifically, the genetically modified succinate-producing yeast strain has a weakened or inactivated gene encoding orotidine 5'-phosphate decarboxylase. Preferably, the gene encoding orotidine 5'-phosphate decarboxylase in the genetically modified succinate-producing yeast strain (for example *Pichia kudriavzevii*) is knocked out.

In one embodiment, the orotidine 5'-phosphate decarboxylase comprises an amino acid sequence of SEQ ID NO: 13, or an amino acid sequence having at least 75%, 80%, 85%, 90%, 91%, 92%, 93%, 94%, 95%, 96%, 97%, 98%, 99% or more identity thereto and having orotidine 5'-phosphate decarboxylase activity.

In one embodiment, the gene encoding the orotidine 5'-phosphate decarboxylase encodes a protein of SEQ ID NO: 13, or an amino acid sequence having at least 75%, 80%, 85%, 90%, 91%, 92%, 93%, 94%, 95%, 96%, 97%, 98%, 99% or more identity thereto and having orotidine 5'-phosphate decarboxylase activity.

In one embodiment, the genetically modified succinate-producing yeast strain further has an activity-reduced or inactivated monocarboxylate permease. Specifically, the genetically modified succinate-producing yeast strain has a weakened or inactivated gene encoding the monocarboxylate permease. Preferably, the gene encoding monocarboxylate permease in the genetically modified succinate-producing yeast strain (for example *Pichia kudriavzevii*) is knocked out.

In one embodiment, the monocarboxylate permease comprises an amino acid sequence of SEQ ID NO: 16, or an amino acid sequence having at least 75%, 80%, 85%, 90%, 91%, 92%, 93%, 94%, 95%, 96%, 97%, 98%, 99% or more identity thereto and having monocarboxylate permease activity.

In one embodiment, the gene encoding the monocarboxylate permease encodes for example a protein of SEQ ID NO: 16, or an amino acid sequence having at least 75%, 80%, 85%, 90%, 91%, 92%, 93%, 94%, 95%, 96%, 97%, 98%, 99% or more identity thereto and having monocarboxylate permease activity.

In one embodiment, the genetically modified succinate-producing yeast strain further has an activity-reduced or inactivated dicarboxylate transport protein. Specifically, the genetically modified succinate-producing yeast strain has a weakened or inactivated gene encoding dicarboxylate transport protein. Preferably, the gene encoding dicarboxylate transport protein in the genetically modified succinate-producing yeast strain (for example *Pichia kudriαvzevii*), for example *JEN2-1* or *JEN2-2* gene, is knocked out.

In one embodiment, the dicarboxylate transport protein comprises an amino acid sequence of SEQ ID NO: 14 or 15, or an amino acid sequence having at least 75%, 80%, 85%, 90%, 91%, 92%, 93%, 94%, 95%, 96%, 97%, 98%, 99% or more identity thereto and having dicarboxylate transport protein activity.

In one embodiment, the gene encoding the dicarboxylate transport protein encodes for example a protein of SEQ ID NO: 14 or 15, or an amino acid sequence having at least 75%, 80%, 85%, 90%, 91%, 92%, 93%, 94%, 95%, 96%, 97%, 98%, 99% or more identity thereto and having dicarboxylate transport protein activity.

In one embodiment, the present invention provides a genetically modified succinate-producing yeast strain (for example *Pichia kudriavzevii,* such as CY902 strain), having an over-expressed *MDH* gene encoding NADPH-dependent malate dehydrogenase, and optionally wherein an endogenous gene encoding orotidine 5'-phosphate decarboxylase and/or an endogenous gene encoding bifunctional enzyme of oxaloacetate decarboxylase and 3-hydroxy-3-methylglutarate aldolase is knocked out. Preferably, said *MDH* gene comprises the sequence of SEQ ID NO: 1 or a degenerate sequence thereof.

In one embodiment, the present invention provides a genetically modified succinate-producing yeast strain (for example *Pichia kudriavzevii,* such as CY902 strain), having an over-expressed *MDH* gene encoding NADPH-dependent malate dehydrogenase and an over-expressed gene encoding soluble fumarate reductase, and optionally wherein an endogenous gene encoding orotidine 5'-phosphate decarboxylase and/or an endogenous gene encoding alcohol dehydrogenase 1 and/or an endogenous gene encoding bifunctional enzyme of oxaloacetate decarboxylase and 3-hydroxy-3-methylglutarate aldolase is knocked out. Preferably, said *MDH* gene comprises the sequence of SEQ ID NO: 1 or a degenerate sequence thereof, or said gene encoding soluble fumarate reductase comprises a sequence of any one of SEQ ID NOs: 3-5 or a degenerate sequence thereof.

In one embodiment, the present invention provides a genetically modified succinate-producing yeast strain (for example *Pichia kudriavzevii,* such as CY902 strain), having an over-expressed *MDH* gene encoding NADPH-dependent malate dehydrogenase and an over-expressed gene encoding succinate transport protein for example *SpMAE1* gene, and optionally wherein an endogenous gene encoding orotidine 5'-phosphate decarboxylase and/or an endogenous gene encoding monocarboxylate permease and/or an endogenous gene encoding bifunctional enzyme of oxaloacetate decarboxylase and 3-hydroxy-3-methylglutarate aldolase is knocked out. Preferably, said *MDH* gene comprises the sequence of SEQ ID NO: 1 or a degenerate sequence thereof, or said gene encoding succinate transport protein comprises the sequence of SEQ ID NO: 2 or a degenerate sequence thereof.

In one embodiment, the present invention provides a genetically modified succinate-producing yeast strain (for example *Pichia kudriavzevii,* such as CY902 strain), having an over-expressed *MDH* gene encoding NADPH-dependent malate dehydrogenase and an over-expressed gene encoding fumarase, and optionally wherein an endogenous gene encoding orotidine 5'-phosphate decarboxylase and/or an endogenous gene encoding pyruvate decarboxylase and/or an endogenous gene encoding bifunctional enzyme of oxaloacetate decarboxylase and 3-hydroxy-3-methylglutarate aldolase is knocked out. Preferably, said *MDH* gene comprises the sequence of SEQ ID NO: 1 or a degenerate sequence thereof, or sad gene encoding fumarase comprises the sequence of SEQ ID NO: 8 or a degenerate sequence thereof.

In one embodiment, the present invention provides a genetically modified succinate-producing yeast strain (for example *Pichia kudriavzevii,* such as CY902 strain), having an over-expressed *MDH* gene encoding NADPH-dependent malate dehydrogenase and an over-expressed gene encoding pyruvate carboxylase, and optionally wherein an endogenous gene encoding orotidine 5'-phosphate decarboxylase and/or an endogenous gene encoding dicarboxylate transport protein such as *JEN2-1* or *JEN2-2* gene and/or an endogenous gene encoding bifunctional enzyme of oxaloacetate decarboxylase and 3-hydroxy-3-methylglutarate aldolase is knocked out. Preferably, said *MDH* gene comprises the sequence of SEQ ID NO: 1 or a degenerate sequence thereof, or said gene encoding pyruvate carboxylase comprises the sequence of SEQ ID NO: 6 or 7 or a degenerate sequence thereof.

In one embodiment, the present invention provides a genetically modified succinate-producing yeast strain (for example *Pichia kudriavzevii,* such as CY902 strain), having an over-expressed *MDH* gene encoding NADPH-dependent malate dehydrogenase and a gene encoding pyruvate carboxylase, and an endogenous gene encoding pyruvate decarboxylase and an endogenous gene encoding NAD-dependent glycerol 3-phosphate dehydrogenase are knocked out, and optionally wherein an endogenous gene encoding orotidine 5'-phosphate decarboxylase and/or an endogenous gene encoding dicarboxylate transport protein (for example a *JEN2* gene, such as *JEN2-1* gene, *JEN2-2* gene) and/or an endogenous gene encoding bifunctional enzyme of oxaloacetate decarboxylase and 3-hydroxy-3-methylglutarate aldolase is knocked out. Preferably, said *MDH* gene comprises the sequence of SEQ ID NO: 1 or a degenerate sequence thereof, or said gene encoding pyruvate carboxylase comprises the sequence of SEQ ID NO: 6 or 7 or a degenerate sequence thereof.

In one embodiment, the present invention provides a genetically modified succinate-producing yeast strain (for example *Pichia kudriavzevii,* such as CY902 strain), having an over-expressed *MDH* gene encoding NADPH-dependent malate dehydrogenase, a gene encoding pyruvate carboxylase, a gene encoding soluble fumarate reductase and a gene encoding succinate transport protein for example *SpMAE1* gene, and wherein an endogenous gene encoding pyruvate decarboxylase and an endogenous gene encoding NAD-dependent glycerol 3-phosphate dehydrogenase are knocked out, and optionally wherein, an endogenous gene encoding orotidine 5'-phosphate decarboxylase and/or an endogenous gene encoding dicarboxylate transport protein (for example a *JEN2* gene, such as *JEN2-1* gene, *JEN2-2* gene) and/or an endogenous gene encoding bifunctional enzyme of oxaloacetate decarboxylase and 3-hydroxy-3-methylglutarate aldolase and/or an endogenous gene encoding alcohol dehydrogenase 1 and/or an endogenous gene encoding monocarboxylate permease is knocked out. Preferably, said *MDH* gene comprises the sequence of SEQ ID NO: 1 or a degenerate sequence thereof, said gene encoding pyruvate carboxylase comprises the sequence of SEQ ID NO: 6 or 7 or a degenerate sequence thereof, said gene encoding soluble fumarate reductase comprises a sequence of any one of SEQ ID Nos: 3-5 or a degenerate sequence thereof, or said gene encoding succinate transport protein comprises the sequence of SEQ ID NO: 2 or a degenerate sequence thereof.

In one embodiment, the present invention provides a genetically modified succinate-producing yeast strain (for example *Pichia kudriavzevii,* such as CY902 strain), having an over-expressed *MDH* gene encoding NADPH-dependent malate dehydrogenase, a gene encoding pyruvate carboxylase, a gene encoding soluble fumarate reductase, a gene encoding fumarase and a gene encoding succinate transport protein for example *SpMAE1* gene, and wherein an endogenous gene encoding pyruvate decarboxylase and an endogenous gene encoding NAD-dependent glycerol 3-phosphate dehydrogenase are knocked out, and optionally wherein an endogenous gene encoding orotidine 5'-phosphate decarboxylase, and/or an endogenous gene encoding dicarboxylate transport protein (for example a *JEN2* gene, such as *JEN2-1* gene, *JEN2-2* gene), and/or an endogenous gene encoding bifunctional enzyme of oxaloacetate decarboxylase and 3-hydroxy-3-methylglutarate aldolase, and/or an endogenous gene encoding alcohol dehydrogenase 1, and/or an endogenous gene encoding a monocarboxylate permease is knocked out. Preferably, said *MDH* gene comprises the sequence of SEQ ID NO: 1 or a degenerate sequence thereof, said gene encoding pyruvate carboxylase comprises the sequence of SEQ ID NO: 6 or 7 or a degenerate sequence thereof, said gene encoding soluble fumarate reductase comprises a sequence of any one of SEQ ID Nos: 3-5 or a degenerate sequence thereof, said gene encoding fumarase comprises the sequence of SEQ ID NO: 8 or a degenerate sequence thereof, or said gene encoding succinate transport protein comprises the sequence of SEQ ID NO: 2 or a degenerate sequence thereof.

In one embodiment, the *JEN2-1* gene encodes a protein of SEQ ID NO: 14, or an amino acid having at least 75%, 80%, 85%, 90%, 91%, 92%, 93%, 94%, 95%, 96%, 97%, 98%, 99% or more identity thereto and having dicarboxylate transport protein activity.

In one embodiment, the *JEN2-2* gene encodes a protein of SEQ ID NO: 15, or an amino acid having at least 75%, 80%, 85%, 90%, 91%, 92%, 93%, 94%, 95%, 96%, 97%, 98%, 99% or more identity thereto and having dicarboxylate transport protein activity.

In one embodiment, the present invention provides a genetically modified *Pichia kudriavzevii* such as CY902 strain, having an over-expressed *MDH* gene encoding NADPH-dependent malate dehydrogenase, and optionally wherein the endogenous *URA3* gene and/or the endogenous *Pk2365* gene is knocked out. Preferably, said *MDH* gene comprises the sequence of SEQ ID NO: 1 or a degenerate sequence thereof.

In one embodiment, the present invention provides a genetically modified *Pichia kudriavzevii* such as CY902 strain, having an over-expressed *MDH* gene encoding NADPH-dependent malate dehydrogenase and an over-expressed gene encoding soluble fumarate reductase, and optionally wherein the endogenous *URA3* gene and/or the endogenous *ADH1* gene and/or the endogenous *Pk2365* gene is knocked out. Preferably, said *MDH* gene comprises the sequence of SEQ ID NO: 1 or a degenerate sequence thereof, or said gene encoding soluble fumarate reductase comprises a sequence of any one of SEQ ID NOs: 3-5 or a degenerate sequence thereof.

In one embodiment, the present invention provides a genetically modified *Pichia kudriavzevii* such as CY902 strain, having an over-expressed *MDH* gene encoding NADPH-dependent malate dehydrogenase and an over-expressed gene encoding a succinate transport protein for example *SpMAE1* gene, and optionally wherein the endogenous *URA3* gene and/or the endogenous *MCH4* gene and/or the endogenous *Pk2365* gene is knocked out. Preferably, said *MDH* gene comprises the sequence of SEQ ID NO: 1 or a degenerate sequence thereof, or said gene encoding succinate transport protein comprises the sequence of SEQ ID NO: 2 or a degenerate sequence thereof.

In one embodiment, the present invention provides a genetically modified *Pichia kudriavzevii* such as CY902 strain, having an over-expressed *MDH* gene encoding NADPH-dependent malate dehydrogenase and an over-expressed gene encoding fumarase, and optionally wherein the endogenous *URA3* gene and/or the endogenous *PDC1* gene and/or the endogenous *Pk2365* gene is knocked out. Preferably, said *MDH* gene comprises the sequence of SEQ ID NO: 1 or a degenerate sequence thereof, or said gene encoding fumarase comprises the sequence of SEQ ID NO: 8 or a degenerate sequence thereof.

In one embodiment, the present invention provides a genetically modified *Pichia kudriavzevii* such as CY902 strain, having an over-expressed *MDH* gene encoding NADPH-dependent malate dehydrogenase and an over-expressed gene encoding pyruvate carboxylase, optionally wherein the endogenous *URA3* gene and/or the endogenous *JEN2-1* or *JEN2-2* gene and/or the endogenous *Pk2365* gene is knocked out. Preferably, said *MDH* gene comprises the sequence of SEQ ID NO: 1 or a degenerate sequence thereof, or said gene encoding pyruvate carboxylase comprises the sequence of SEQ ID NO: 6 or 7 or a degenerate sequence thereof.

In one embodiment, the present invention provides a genetically modified *Pichia kudriavzevii* such as CY902 strain, having an over-expressed *MDH* gene encoding NADPH-dependent malate dehydrogenase and an over-expressed gene encoding pyruvate carboxylase, wherein the endogenous *PDC1* gene and the endogenous *GPD1* gene are knocked out, and optionally wherein the endogenous *URA3* gene, and/or an endogenous *JEN2* gene, such as *JEN2-1* gene, *JEN2-2* gene, and/or the endogenous *Pk2365* gene is knocked out. Preferably, said *MDH* gene comprises the sequence of SEQ ID NO: 1 or a degenerate sequence thereof, or said gene encoding pyruvate carboxylase comprises the sequence of SEQ ID NO: 6 or 7 or a degenerate sequence thereof.

In one embodiment, the present invention provides a genetically modified *Pichia kudriavzevii* such as CY902 strain, having an over-expressed *MDH* gene encoding NADPH-dependent malate dehydrogenase, an over-expressed gene encoding pyruvate carboxylase, a gene encoding soluble fumarate reductase and a gene encoding succinate transport protein for example *SpMAE1* gene, wherein the endogenous *PDC1* gene and the endogenous *GPD1* gene are knocked out, and optionally wherein the endogenous *URA3* gene and/or the endogenous *JEN2* gene such as *JEN2-1* gene, *JEN2-2* gene and/or the endogenous *Pk2365* gene and/or the endogenous *ADH1* gene and/or the endogenous *MCH4* gene is knocked out. Preferably, said *MDH* gene comprises the sequence of SEQ ID NO: 1 or a degenerate sequence thereof, said gene encoding pyruvate carboxylase comprises the sequence of SEQ ID NO: 6 or 7 or a degenerate sequence thereof, said gene encoding soluble fumarate reductase comprises a sequence of any one of SEQ ID Nos: 3-5 or a degenerate sequence thereof, or said gene encoding succinate transport protein comprises the sequence of SEQ ID NO: 2 or a degenerate sequence thereof.

In one embodiment, the present invention provides a genetically modified *Pichia kudriavzevii* such as CY902 strain, having activity-enhanced *Sorghum bicolor* NADPH-dependent malate dehydrogenase, *Aspergillus oryzae* pyruvate carboxylase, *Trypanosoma brucei* soluble fumarate reductase, *Pichia kudriavzevii* fumarase (the mitochondrion-localized peptide at the 5'-end thereof is truncated) and succinate transport protein SpMAE1, wherein the endogenous pyruvate decarboxylase and the endogenous NAD-dependent glycerol 3-phosphate dehydrogenase activities are eliminated. In one embodiment, the present invention provides a genetically modified *Pichia kudriavzevii* such as CY902 strain, having an over-expressed *MDH* gene encoding NADPH-dependent malate dehydrogenase, a gene encoding pyruvate carboxylase, a gene encoding soluble fumarate reductase, a gene encoding fumarase and a gene encoding a succinate transport protein such as *SpMAE1* gene, wherein the endogenous *PDC1* gene and the endogenous *GPD1* gene are knocked out, and optionally wherein the endogenous *URA3* gene, and/or the endogenous *JEN2* gene such as *JEN2-1* gene, *JEN2-2* gene, and/or the endogenous *Pk2365* gene and/or the endogenous *ADH1* gene, and/or the endogenous *MCH4* gene is knocked out. Preferably, said *MDH* gene comprises the sequence of SEQ ID NO: 1 or a degenerate sequence thereof, or said gene encoding pyruvate carboxylase comprises the sequence of SEQ ID NO: 6 or 7 or a degenerate sequence thereof, said gene encoding soluble fumarate reductase comprises a sequence of any one of SEQ ID Nos: 3-5 or a degenerate sequence thereof, said gene encoding fumarase comprises the sequence of SEQ ID NO: 8 or a degenerate sequence thereof, or said gene encoding succinate transport protein comprises the sequence of SEQ ID NO: 2 or a degenerate sequence thereof.

In a further aspect, the present invention provides a method for constructing a genetically modified succinate-producing yeast strain, comprising imparting NADPH-dependent malate dehydrogenase (EC 1.1.1.82) activity to the strain or enhancing NADPH-dependent malate dehydrogenase (EC 1.1.1.82) activity in the strain.

As used herein, "imparting ... activity" refers to producing an activity in the genetically modified succinate-producing yeast strain, which was not present in the initial strain prior to genetic modification. As used herein, "enhancing...activity (of)" refers to increasing the activity for example by at least 5%, at least 10%, at least 20%, at least 30%, at least 40%, at least 50%, at least 60%, at least 70%, at least 80%, at least 90%, at least 95%, at least 100%, at least 150%, at least 200%, at least 250%, at least 300% or more.

There are many methods known in the art for imparting or enhancing the desired protein activity, for example including but not limited to expressing or over-expressing a protein-encoding gene as well as the mutation or any other modification that increases the protein activity.

As used herein, "over-expressing" means that the gene expression level is increased compared to the level prior to genetic manipulation, for example increased by at least 5%, at least 10%, at least 20%, at least 30%, at least 40%, at least 50%, at least 60%, at least 70%, at least 80%, at least 90%, at least 95%, at least 100%, at least 150%, at least 200%, at least 250%, at least 300% or more. Methods for over-expressing a gene are well-known in the art, for example including but not limited to using a strong promoter, increased gene copy number, an enhancer, etc. The increased gene copy number can be achieved by for example, but not limited to, introducing one or more copies of an exogenous gene or an endogenous gene, for example through an expression vector or integration into a genome.

As used herein, the "exogenous gene" refers to a gene from another cell or organism, for example a gene from the same species or a different species.

As used herein, the "endogenous gene" refers to a gene from a cell or organism itself.

The promoter can be selected from any suitable promoters well-known in the art, for example including, but not limited to, the promoter of FBA1 gene encoding fructose 1,6-biphosphate aldolase, the promoter of *TDH3* gene encoding glyceraldehyde 3-phosphate dehydrogenase, the promoter of *PDC1* gene encoding pyruvate decarboxylase, the promoter of *ADH1* gene encoding alcohol dehydrogenase 1, the promoter of *PGK1* gene encoding 3-phosphoglycerate kinase, the promoter of *TEF1* gene encoding a transcription elongation factor, the promoter of *GPM1* gene encoding phosphoglycerate mutase, the promoter of *TPI1* gene encoding triose phosphate isomerase and the promoter of *ENO1* gene encoding enolase (as set forth in SEQ ID NO: 100).

In one embodiment, the NADPH-dependent malate dehydrogenase is derived from plants, preferably C4 plants, more preferably Gramineae, Cyperaceae, Compositae, Euphorbiaceae, Chenopodiaceae, Portulacaceae and Amaranthaceae plants, or is derived from *Euglenα* or *Thermobacillus,* more preferably *Sorghum bicolor, Zea mays, Saccharum officinαrum, Pisum sativum, Cicer arietinum, Spinαciα oleracea, Euglena gracilis* or *Methanothermobacter thermautotrophicus,* more preferably *Sorghum bicolor.*

In one embodiment, the NADPH-dependent malate dehydrogenase activity is generated or increased by expressing or over-expressing an *MDH* gene encoding NADPH-dependent malate dehydrogenase in the genetically modified succinate-producing yeast strain. Preferably, said *MDH* gene encoding NADPH-dependent malate dehydrogenase is derived from plants, preferably C4 plants, more preferably Gramineae, Cyperaceae, Compositae, Euphorbiaceae, Chenopodiaceae, Portulacaceae and Amaranthaceae plants, or is derived from *Euglenα* and *Thermobacillus,* more preferably *Sorghum bicolor, Zea mays, Saccharum officinarum,* Pisum sativum, *Cicer arietinum, Spinacia oleracea, Euglena gracilis* or *Methanothermobacter thermautotrophicus,* more preferably *Sorghum bicolor.*

In one embodiment, said *MDH* gene encoding NADPH-dependent malate dehydrogenase comprises the sequence of SEQ ID NO: 1 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having NADPH-dependent malate dehydrogenase activity.

In one embodiment, said *MDH* gene encoding NADPH-dependent malate dehydrogenase is incorporated into the genome of the genetically modified succinate-producing yeast (for example *Pichia kudriαvzevii),* for example at a position of *Pk2365* loci. In one embodiment, said *MDH* gene encoding NADPH-dependent malate dehydrogenase is incorporated into the genome of the yeast (for example *Pichia kudriαvzevii)* via homologous recombination, for example at a position of *Pk2365* loci. Said MDH gene encoding NADPH-dependent malate dehydrogenase can be placed under the control of a suitable promoter (for example the promoter of *FBA1* gene (as set forth in SEQ ID NO: 17)) and/or a terminator (for example the terminator of *INO1* gene (as set forth in SEQ ID NO: 18)).

In a further embodiment, the method further comprises imparting a soluble fumarate reductase activity to the strain or enhancing the soluble fumarate reductase activity in the strain.

In one embodiment, said soluble fumarate reductase is derived from yeasts and the order Kinetoplastida, for example but is not limited to *Saccharomyces cerevisiae, Trypanosoma brucei, Leishmania mexicana, Trypanosoma cruzi.*

In one embodiment, the glyoxysome-localized peptide at the 3' end of the soluble fumarate reductase is partially or completely truncated, so that it is freely present in the cytoplasm.

In one embodiment, the soluble fumarate reductase activity is generated or increased by expressing or over-expressing a gene encoding soluble fumarate reductase in the genetically modified succinate-producing yeast strain. Preferably, said gene encoding soluble fumarate reductase is preferably derived from yeasts and the order Kinetoplastida, for example including but not limited to *Saccharomyces cerevisiae, Trypanosoma brucei, Leishmania mexicana* and *Trypanosoma cruzi.*

In one embodiment, said gene encoding soluble fumarate reductase comprises a sequence of any one of SEQ ID Nos: 3-5 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having fumarate reductase activity.

In one embodiment, the gene encoding soluble fumarate reductase is incorporated into the genome of the genetically modified succinate-producing yeast (for example *Pichia kudriavzevii*), for example at a position of *ADH1* loci. The gene encoding soluble fumarate reductase can be placed under the control of a suitable promoter (for example the promoter of *ADH1* gene (as set forth in SEQ ID NO: 21)) and/or a terminator (for example the terminator of *ADH1* gene (as set forth in SEQ ID NO: 22)).

In a further embodiment, the method further comprises imparting or enhancing succinate transport protein activity in the yeast strain.

In one embodiment, said succinate transport protein is selected from a group consisting of SpMAE1 protein, AnDCT-02 protein, EcDcuB protein and EcDcuC protein. In one embodiment, the succinate transport protein activity is imparted or enhanced by expressing or over-expressing a gene encoding succinate transport protein (for example *SpMAE1* gene) in the genetically modified succinate-producing yeast strain.

In one embodiment, said *SpMAE1* gene comprises the sequence of SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having succinate transport protein activity, and optionally is (for example via homologous recombination) incorporated into the genome of the genetically modified succinate-producing yeast strain (for example *Pichia kudriavzevii*), for example at a position of *MCH4* loci. Said *SpMAE1* gene can be placed under the control of a suitable promoter (for example the promoter of *TDH3* gene (as set forth in SEQ ID NO: 19)) and/or a terminator (for example the terminator of *GAL2* gene (as set forth in SEQ ID NO: 20)).

In a further embodiment, the method further comprises imparting or enhancing fumarase activity in the yeast strain.

In one embodiment, a mitochondrion-localized peptide at the 5' end of said fumarase is partially or completely truncated, so that it cannot localize to the mitochondrion but is freely present in the cytoplasm.

In one embodiment, the fumarase activity is imparted or enhanced by expressing or over-expressing a gene encoding fumarase in the genetically modified succinate-producing yeast strain. In one embodiment, said fumarase is derived from for example but not limited to *Actinobacillus succinogenes, Mannheimia succiniciproducens, Escherichia coli, Pichia kudriαvzevii, Rhizopus oryzae.*

In one embodiment, the fumarase comprises the amino acid sequence of SEQ ID NO: 95, or an amino acid having at least 75%, 80%, 85%, 90%, 91%, 92%, 93%, 94%, 95%, 96%, 97%, 98%, 99% or more identity thereto and having fumarase activity.

In one embodiment, the gene encoding the fumarase encodes the amino acid sequence of SEQ ID NO: 95, or an amino acid sequence having at least 75%, 80%, 85%, 90%, 91%, 92%, 93%, 94%, 95%, 96%, 97%, 98%, 99% or more identity thereto and having fumarase activity.

In one embodiment, the gene encoding the fumarase comprises the sequence of SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having fumarase activity.

Optionally, the gene encoding the fumarase is incorporated (for example via homologous recombination) into the genome of the genetically modified succinate-producing yeast (for example *Pichia kudriavzevii),* for example at a position of *PDC1* loci. The gene encoding the fumarase can be placed under the control of a suitable promoter (for example the promoter of *TDH3* gene (as set forth in SEQ ID NO: 19)) and/or a terminator (for example the terminator of *GAL2* gene (as set forth in SEQ ID NO: 20)).

In a further embodiment, the method further comprises imparting or enhancing pyruvate carboxylase activity in the yeast strain. Preferably, imparting or enhancing pyruvate carboxylase activity is achieved by expressing or over-expressing a gene encoding pyruvate carboxylase in the strain. The pyruvate carboxylase can be derived from fungi, especially yeasts and filamentous fungi, preferably *Saccharomyces cerevisiae, Pichia kudriavzevii, Aspergillus oryzae, Kluyveromyces marxianus*, etc. In one embodiment, the gene encoding pyruvate carboxylase can be selected from a group consisting of the *PYC* gene of *Aspergillus oryzae* and the *PYC1* gene of *Pichia kudriavzevii.*

In one embodiment, said pyruvate carboxylase comprises an amino acid sequence encoded by the sequence of SEQ ID NO: 6 or 7, or an amino acid sequence having at least 75%, 80%, 85%, 90%, 91%, 92%, 93%, 94%, 95%, 96%, 97%, 98%, 99% or more identity thereto and having pyruvate carboxylase activity.

In one embodiment, the gene encoding pyruvate carboxylase comprises the sequence of SEQ ID NO: 6 or 7 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity.

Optionally, the gene encoding pyruvate carboxylase is incorporated into the genome of the genetically modified succinate-producing yeast (for *example Pichia kudriavzevii*), for example at a position of *JEN2-1* loci. The gene encoding the pyruvate carboxylase can be placed under the control of a suitable promoter (for example the promoter of *TDH3* gene (as set forth in SEQ ID NO: 19)) and/or a terminator (for example the terminator of *GAL2* gene (as set forth in SEQ ID NO: 20)).

By the method, the gene to be expressed or over-expressed in the strain can be integrated into a suitable position in the strain genome, as long as such integration does not negatively affect the growth, proliferation and/or productivity of the strain. For example, the method comprises integrating one or more of the above-mentioned genes at any one or more positions of the genomes encoding the following proteins: (i) pyruvate decarboxylase (EC 4.1.1.43), (ii) NAD-dependent glycerol 3-phosphate dehydrogenase (EC 1.1.1.8), (iii) orotidine 5'-phosphate decarboxylase (EC 4.1.1.23), (iv) monocarboxylate permease, (v) dicarboxylate transport protein, (vi) alcohol dehydrogenase 1 (EC 1.1.1.1), and (vii) bifunctional enzyme of oxaloacetate decarboxylase and 3-hydroxy-3-methylglutarate aldolase (EC 4.1.3.17 or 4.1.1.112).

In a further embodiment, the method further comprises weakening or inactivating at least one of the following: (i) pyruvate decarboxylase (EC 4.1.1.43), (ii) NAD-dependent glycerol 3-phosphate dehydrogenase (EC 1.1.1.8), (iii) orotidine 5'-phosphate decarboxylase (EC 4.1.1.23), (iv) monocarboxylate permease, (v) dicarboxylate transport protein, (vi) alcohol dehydrogenase 1 (EC 1.1.1.1), and (vii) bifunctional enzyme of oxaloacetate decarboxylase and 3-hydroxy-3-methylglutarate aldolase (EC 4.1.3.17 or 4.1.1.112), in the strain.

In a further embodiment, said method further comprises reducing or inactivating pyruvate decarboxylase activity in the yeast strain.

As used herein, reducing or inactivating the activity of a protein such as an enzyme means that the activity of the protein is reduced by at least 5%, at least 10%, at least 20%, at least 30%, at least 40%, at least 50%, at least 60%, at least 70%, at least 80%, at least 90%, at least 95%, at least 99% or more, or even is undetectable. There are various means in the art for the reducing and inactivating, including, for example, inhibition of gene expression such as knockdown (for example using a small interfering RNA), use of a weak promoter (when the gene is a polypeptide-encoding gene), etc; gene knockout and deletion of partial or compelete sequence of a gene or a polypeptide; mutation of certain loca such as an encoding sequence or an activity domain in a gene or a polypeptide to reduce the gene expression or regulate the activity or express the activity of a product; and use of an antagonist or an inhibitor (for example including but not limited to an antibody, interfering RNA, etc).

As used herein, weakening or inactivating a gene means that the expression level (when being used as a protein-encoding gene) or regulatory performance (when being used as a regulator element) of the gene is reduded by at least 5%, at least 10%, at least 20%, at least 30%, at least 40%, at least 50%, at least 60%, at least 70%, at least 80%, at least 90%, at least 95%, at least 99% or more, or even is undetectable. There are various means in the art for weakening or inactivating a gene, including, for example, inhibition of gene expression such as knockdown (for example using a small interfering RNA), use of a weak promoter (when the gene is a polypeptide-encoding gene), etc; gene knockout and deletion of partial or all gene sequences; mutation of certain loca such as an encoding sequence in a gene to reduce the gene expression or regulate the activity or express the activity of a product, etc.

In one embodiment, reducing or inactivating pyruvate decarboxylase activity includes weakening or inactivating the gene encoding pyruvate decarboxylase.

In one embodiment, weakening or inactivating the gene encoding pyruvate decarboxylase comprises knocking out the gene encoding pyruvate decarboxylase (for example an amino acid sequence of SEQ ID NO: 10, or an amino acid sequence having at least 75%, 80%, 85%, 90%, 91%, 92%, 93%, 94%, 95%, 96%, 97%, 98%, 99% or more identity thereto and having pyruvate decarboxylase activity).

In a further embodiment, the method further comprises reducing or inactivating NAD-dependent glycerol 3-phosphate dehydrogenase activity in the yeast strain.

In one embodiment, reducing or inactivating NAD-dependent glycerol 3-phosphate dehydrogenase activity comprises weakening or inactivating the gene encoding NAD-dependent glycerol 3-phosphate dehydrogenase. In one embodiment, weakening or inactivating the gene encoding NAD-dependent glycerol 3-phosphate dehydrogenase comprises knocking out the gene encoding NAD-dependent glycerol 3-phosphate dehydrogenase (for example encoding an amino acid sequence of SEQ ID NO: 11, or an amino acid sequence having at least 75%, 80%, 85%, 90%, 91%, 92%, 93%, 94%, 95%, 96%, 97%, 98%, 99% or more identity thereto and having NAD-dependent glycerol 3-phosphate dehydrogenase activity).

In a further embodiment, the method further comprises reducing or inactivating the activity (activities) of orotidine 5'-phosphate decarboxylase and/or alcohol dehydrogenase 1 and/or monocarboxylate permease and/or dicarboxylate transport protein and/or bifunctional enzyme of oxaloacetate decarboxylase and 3-hydroxy-3-methylglutarate aldolase in the yeast strain.

In one embodiment, the method comprises weakening or inactivating one or more of the gene encoding orotidine 5'-phosphate decarboxylase, the gene encoding dicarboxylate transport protein (for example a *JEN2* gene such as *JEN2-1* gene, *JEN2-2* gene), the gene encoding alcohol dehydrogenase 1, the gene encoding monocarboxylate permease, and the gene encoding bifunctional enzyme of oxaloacetate decarboxylase and 3-hydroxy-3-methylglutarate aldolase in the yeast strain.

In one embodiment, the gene encoding bifunctional enzyme of oxaloacetate decarboxylase and 3-hydroxy-3-methylglutarate aldolase (for example encoding the amino acid sequence of SEQ ID NO: 9, or an amino acid sequence having at least 75%, 80%, 85%, 90%, 91%, 92%, 93%, 94%, 95%, 96%, 97%, 98%, 99% or more identity thereto and having activity of bifunctional enzyme of oxaloacetate decarboxylase and 3-hydroxy-3-methylglutarate aldolase) is knocked out, for example through homologous recombination.

In one embodiment, the gene encoding orotidine 5'-phosphate decarboxylase (which encodes the amino acid sequence of SEQ ID NO: 13, or an amino acid sequence having at least 75%, 80%, 85%, 90%, 91%, 92%, 93%, 94%, 95%, 96%, 97%, 98%, 99% or more identity thereto and having orotidine 5'-phosphate decarboxylase activity) is knocked out, for example through homologous recombination.

In one embodiment, the *JEN2-1* gene encoding dicarboxylate transport protein (encoding for example the amino acid sequence of SEQ ID NO: 14, or an amino acid sequence having at least 75%, 80%, 85%, 90%, 91%, 92%, 93%, 94%, 95%, 96%, 97%, 98%, 99% or more identity thereto and having dicarboxylate transport protein activity) is knocked out, for example through homologous recombination.

In one embodiment, the gene *JEN2-2* gene encoding dicarboxylate transport protein (encoding for example the amino acid sequence of SEQ ID NO: 15, or an amino acid sequence having at least 75%, 80%, 85%, 90%, 91%, 92%, 93%, 94%, 95%, 96%, 97%, 98%, 99% or more identity thereto and having dicarboxylate transport protein activity) is knocked out, for example through homologous recombination.

In one embodiment, the gene encoding alcohol dehydrogenase 1 (which encodes the amino acid sequence of SEQ ID NO: 12, or an amino acid sequence having at least 75%, 80%, 85%, 90%, 91%, 92%, 93%, 94%, 95%, 96%, 97%, 98%, 99% or more identity thereto and having alcohol dehydrogenase 1 activity) is knocked out, for example through homologous recombination.

In one embodiment, the gene encoding monocarboxylate permease (encoding for example the amino acid sequence of SEQ ID NO: 16, or an amino acid having at least 75%, 80%, 85%, 90%, 91%, 92%, 93%, 94%, 95%, 96%, 97%, 98%, 99% or more identity thereto and having monocarboxylate permease activity) is knocked out, for example through homologous recombination.

In one embodiment, the present invention provides a method for producing a genetically modified succinate-producing yeast strain (for example *Pichia kudriαvzevii,* such as CY902), comprising over-expressing an *MDH* gene encoding NADPH-dependent malate dehydrogenase in the genetically modified succinate-producing yeast strain, optionally comprising knocking out an endogenous gene encoding orotidine 5'-phosphate decarboxylase and/or an endogenous gene encoding an bifunctional enzyme of oxaloacetate decarboxylase and 3-hydroxy-3-methylglutarate aldolase.

In one embodiment, the present invention provides a method for producing a genetically modified succinate-producing yeast strain (for example *Pichia kudriαvzevii,* such as CY902), comprising over-expressing an *MDH* gene encoding NADPH-dependent malate dehydrogenase and a gene encoding soluble fumarate reductase, optionally knocking out an endogenous gene encoding orotidine 5'-phosphate decarboxylase and/or an endogenous gene encoding alcohol dehydrogenase 1 and/or an endogenous gene encoding an bifunctional enzyme of oxaloacetate decarboxylase and 3-hydroxy-3-methylglutarate aldolase, in the genetically modified succinate-producing yeast strain.

In one embodiment, the present invention provides a method for producing a genetically modified succinate-producing yeast strain (for example *Pichia kudriαvzevii,* such as CY902), comprising over-expressing an *MDH* gene encoding NADPH-dependent malate dehydrogenase and a gene encoding succinate transport protein such as *SpMAE1* gene, optionally knocking out an endogenous gene encoding orotidine 5'-phosphate decarboxylase and/or an endogenous gene encoding monocarboxylate permease and/or an endogenous gene encoding an bifunctional enzyme of oxaloacetate decarboxylase and 3-hydroxy-3-methylglutarate aldolase, in the genetically modified succinate-producing yeast strain.

In one embodiment, the present invention provides a method for producing a genetically modified succinate-producing yeast strain (for example *Pichia kudriαvzevii,* such as CY902), comprising over-expressing an *MDH* gene encoding NADPH-dependent malate dehydrogenase and a gene encoding fumarase, optionally knocking out an endogenous gene encoding orotidine 5'-phosphate decarboxylase and/or an endogenous gene encoding pyruvate decarboxylase and/or an endogenous gene encoding an bifunctional enzyme of oxaloacetate decarboxylase and 3-hydroxy-3-methylglutarate aldolase, in the genetically modified succinate-producing yeast strain.

In one embodiment, the present invention provides a method for producing a genetically modified succinate-producing yeast strain (for example *Pichia kudriαvzevii,* such as CY902), comprising over-expressing an *MDH* gene encoding NADPH-dependent malate dehydrogenase and a gene encoding pyruvate carboxylase, optionally knocking out an endogenous gene encoding orotidine 5'-phosphate decarboxylase and/or an endogenous gene encoding dicarboxylate transport protein such as *JEN2-1* or *JEN2-2* gene and/or an endogenous gene encoding an bifunctional enzyme of oxaloacetate decarboxylase and 3-hydroxy-3-methylglutarate aldolase, in the genetically modified succinate-producing yeast strain.

In one embodiment, the present invention provides a method for producing a genetically modified succinate-producing yeast strain (for example *Pichia kudriavzevii,* such as CY902), comprising over-expressing an *MDH* gene encoding NADPH-dependent malate dehydrogenase and a gene encoding pyruvate carboxylase, and knocking out an endogenous gene encoding pyruvate decarboxylase and an endogenous gene encoding NAD-dependent glycerol 3-phosphate dehydrogenase, optionally knocking out an endogenous gene encoding orotidine 5'-phosphate decarboxylase and/or an endogenous gene encoding dicarboxylate transport protein (for example a *JEN2* gene such as *JEN2-1* gene, *JEN2-2* gene) and/or an endogenous gene encoding an bifunctional enzyme of oxaloacetate decarboxylase and 3-hydroxy-3-methylglutarate aldolase, in the genetically modified succinate-producing yeast strain.

In one embodiment, the present invention provides a method for producing a genetically modified succinate-producing yeast strain (for example *Pichia kudriavzevii,* such as CY902), comprising over-expressing an *MDH* gene encoding NADPH-dependent malate dehydrogenase, a gene encoding pyruvate carboxylase, a gene encoding soluble fumarate reductase and a gene encoding succinate transport protein such as *SpMAE1* gene and knocking out an endogenous gene encoding pyruvate decarboxylase and an endogenous gene encoding NAD-dependent glycerol 3-phosphate dehydrogenase, optionally knocking out an endogenous gene encoding orotidine 5'-phosphate decarboxylase and/or an endogenous gene encoding dicarboxylate transport protein (for example a *JEN2* gene such as *JEN2-1* gene, *JEN2-2* gene) and/or an endogenous gene encoding an bifunctional enzyme of oxaloacetate decarboxylase and 3-hydroxy-3-methylglutarate aldolase and/or an endogenous gene encoding alcohol dehydrogenase 1 and/or an endogenous gene encoding monocarboxylate permease, in the genetically modified succinate-producing yeast strain. Preferably, said *MDH* gene comprises the sequence of SEQ ID NO: 1 or a degenerate sequence thereof, or said gene encoding pyruvate carboxylase comprises the sequence of SEQ ID NO: 6 or 7 or a degenerate sequence thereof, said gene encoding soluble fumarate reductase comprises a sequence of any one of SEQ ID Nos: 3-5 or a degenerate sequence thereof, said gene encoding fumarase comprises the sequence of SEQ ID NO: 8 or a degenerate sequence thereof, or said gene encoding succinate transport protein comprises the sequence of SEQ ID NO: 2 or a degenerate sequence thereof.

In one embodiment, the present invention provides a method for producing a genetically modified succinate-producing yeast strain (for example *Pichia kudriavzevii,* such as CY902), comprising over-expressing an *MDH* gene encoding NADPH-dependent malate dehydrogenase, a gene encoding pyruvate carboxylase, a gene encoding soluble fumarate reductase, a gene encoding fumarase and a gene encoding succinate transport protein such as *SpMAE1* gene and knocking out an endogenous gene encoding pyruvate decarboxylase and an endogenous gene encoding NAD-dependent glycerol 3-phosphate dehydrogenase, optionally knocking out an endogenous gene encoding orotidine 5'-phosphate decarboxylase and/or an endogenous gene encoding dicarboxylate transport protein (for example a *JEN2* gene such as *JEN2-1* gene, *JEN2-2* gene) and/or an endogenous gene encoding an bifunctional enzyme of oxaloacetate decarboxylase and 3-hydroxy-3-methylglutarate aldolase and/or an endogenous gene encoding alcohol dehydrogenase 1 and/or an endogenous gene encoding monocarboxylate permease, in the genetically modified succinate-producing yeast strain. Preferably, said *MDH* gene comprises the sequence of SEQ ID NO: 1 or a degenerate sequence thereof, said gene encoding pyruvate carboxylase comprises the sequence of SEQ ID NO: 6 or 7 or a degenerate sequence thereof, said gene encoding soluble fumarate reductase comprises a sequence of any one of SEQ ID Nos: 3-5 or a degenerate sequence thereof, said gene encoding fumarase comprises the sequence of SEQ ID NO: 8 or a degenerate sequence thereof, or said gene encoding succinate transport protein comprises the sequence of SEQ ID NO: 2 or a degenerate sequence thereof.

In one embodiment, the present invention provides a method for producing a genetically modified *Pichia kudriavzevii* such as CY902 strain, comprising over-expressing an *MDH* gene encoding NADPH-dependent malate dehydrogenase, optionally knocking out the endogenous *URA3* gene and/or the endogenous *Pk2365* gene. Preferably, said *MDH* gene comprises the sequence of SEQ ID NO: 1 or a degenerate sequence thereof.

In one embodiment, the present invention provides a method for producing a genetically modified *Pichia kudriavzevii* such as CY902 strain, comprising over-expressing an *MDH* gene encoding NADPH-dependent malate dehydrogenase and a gene encoding soluble fumarate reductase, optionally knocking out the endogenous *URA3* gene and/or the endogenous *ADH1* gene and/or the endogenous *Pk2365* gene. Preferably, said *MDH* gene comprises the sequence of SEQ ID NO: 1 or a degenerate sequence thereof, or said gene encoding soluble fumarate reductase comprises a sequence of any one of SEQ ID NOs: 3-5 or a degenerate sequence thereof.

In one embodiment, the present invention provides a method for producing a genetically modified *Pichia kudriavzevii* such as CY902 strain, comprising over-expressing an *MDH* gene encoding NADPH-dependent malate dehydrogenase and a gene encoding succinate transport protein such as *SpMAE1* gene, optionally knocking out the endogenous *URA3* gene and/or the endogenous *MCH4* gene and/or the endogenous *Pk2365* gene. Preferably, said *MDH* gene comprises the sequence of SEQ ID NO: 1 or a degenerate sequence thereof, or said gene encoding succinate transport protein comprises the sequence of SEQ ID NO: 2 or a degenerate sequence thereof.

In one embodiment, the present invention provides a method for producing a genetically modified *Pichia kudriavzevii* such as CY902 strain, comprising over-expressing an *MDH* gene encoding NADPH-dependent malate dehydrogenase and a gene encoding fumarase, optionally knocking out the endogenous *URA3* gene and/or the endogenous *PDC1* gene and/or the endogenous *Pk2365* gene. Preferably, said *MDH* gene comprises the sequence of SEQ ID NO: 1 or a degenerate sequence thereof, or said gene encoding fumarase comprises the sequence of SEQ ID NO: 8 or a degenerate sequence thereof.

In one embodiment, the present invention provides a method for producing a genetically modified *Pichia kudriavzevii* such as CY902 strain, comprising over-expressing an *MDH* gene encoding NADPH-dependent malate dehydrogenase and a gene encoding pyruvate carboxylase, optionally knocking out the endogenous *URA3* gene and/or the endogenous *JEN2-1* or *JEN2-2* gene and/or the endogenous *Pk2365* gene. Preferably, said *MDH* gene comprises the sequence of SEQ ID NO: 1 or a degenerate sequence thereof, or said gene encoding pyruvate carboxylase comprises the sequence of SEQ ID NO: 6 or 7 or a degenerate sequence thereof.

In one embodiment, the present invention provides a method for producing a genetically modified *Pichia kudriavzevii* such as CY902 strain, comprising over-expressing an *MDH* gene encoding NADPH-dependent malate dehydrogenase and a gene encoding pyruvate carboxylase, and knocking out the endogenous *PDC1* gene and the endogenous *GPD1* gene, optionally knocking out the endogenous *URA3* gene and/or the endogenous *JEN2* gene, such as *JEN2-1* gene, *JEN2-2* gene, and/or the endogenous *Pk2365* gene. Preferably, said *MDH* gene comprises the sequence of SEQ ID NO: 1 or a degenerate sequence thereof, or said gene encoding pyruvate carboxylase comprises the sequence of SEQ ID NO: 6 or 7 or a degenerate sequence thereof.

In one embodiment, the present invention provides a method for producing a genetically modified *Pichia kudriavzevii* such as CY902 strain, comprising over-expressing an *MDH* gene encoding NADPH-dependent malate dehydrogenase, a gene encoding pyruvate carboxylase, a gene encoding soluble fumarate reductase and a gene encoding succinate transport protein such as *SpMAE1* gene, and knocking out the endogenous *PDC1* gene and the endogenous *GPD1* gene, optionally knocking out the endogenous *URA3* gene and/or the endogenous *JEN2* gene such as *JEN2-1* gene, *JEN2-2* gene, and/or the endogenous *Pk2365* gene and/or the endogenous *ADH1* gene and/or the endogenous *MCH4* gene. Preferably, said *MDH* gene comprises the sequence of SEQ ID NO:1 or a degenerate sequence thereof, or said gene encoding pyruvate carboxylase comprises the sequence of SEQ ID NO: 6 or 7 or a degenerate sequence thereof, the gene encoding soluble fumarate reductase comprises a sequence of any one of SEQ ID Nos: 3-5 or a degenerate sequence thereof, or said gene encoding succinate transport protein comprises the sequence of SEQ ID NO: 2 or a degenerate sequence thereof.

In one embodiment, the present invention provides a method for producing a genetically modified *Pichia kudriavzevii* such as CY902 strain, comprising over-expressing an *MDH* gene encoding NADPH-dependent malate dehydrogenase, a gene encoding pyruvate carboxylase, a gene encoding soluble fumarate reductase, a gene encoding fumarase and a gene encoding succinate transport protein such as *SpMAE1* gene, and knocking out the endogenous *PDC1* gene and the endogenous *GPD1* gene, optionally knocking out the endogenous *URA3* gene and/or the endogenous *JEN2* gene such as *JEN2-1* gene, *JEN2-2* gene and/or the endogenous *Pk2365* gene and/or the endogenous *ADH1* gene and/or the endogenous *MCH4* gene.

Preferably, said *MDH* gene comprises the sequence of SEQ ID NO: 1 or a degenerate sequence thereof, or said gene encoding pyruvate carboxylase comprises the sequence of SEQ ID NO: 6 or 7 or a degenerate sequence thereof, said gene encoding soluble fumarate reductase comprises a sequence of any one of SEQ ID Nos: 3-5 or a degenerate sequence thereof, or said gene encoding fumarase comprises the sequence of SEQ ID NO: 8 or a degenerate sequence thereof, or said gene encoding succinate transport protein comprises the sequence of SEQ ID NO: 2 or a degenerate sequence thereof.

In one embodiment, the succinate-producing yeast strain to be genetically modified includes but is not limited to the genera *Candida, Pichia, Rhodotroula, Saccharomyces, Yarrowia, Zygosaccharomyces,* and *Torulopsis.* In one embodiment, the succinate-producing yeast strain used to perform genetic modification is selected from a group consisting of *Pichia* spp., *Saccharomyces* spp.or *Yarrowia* spp. In a preferred embodiment, the succinate-producing yeast strain used to perform genetic modification is *Pichia kudriαvzevii, Saccharomyces cerevisiae* or *Yarrowia lipolyticα,* for example the *Pichia kudriavzevii* preserved in China General Microbiological Culture Collection Center (CGMCC) under the deposit number of CGMCC No. 20885.

In one aspect, the present invention provides a method for producing succinate, comprising culturing the genetically modified succinate-producing yeast strain according to the present invention or the genetically modified succinate-producing yeast strain prepared by the method for constructing a genetically modified succinate-producing yeast strain according to the present invention under suitable conditions for the fermentation production of succinate, optionally comprising isolating and purifying the produced succinate.

It is known in the art that the conditions for the fermentation production of succinate, under which the succinate-producing yeast strain is cultured via fermentation, include but are not limited to pH, temperature, culture medium component, fermentation time, etc.

It is known in the art that the culture medium for the fermentation production of succinate by the succinate-producing yeast strain includes but is not limited to an inorganic salt culture medium (about 5-12% w/v glucose, optionally containing 30 g/L CaCOs).

It is known that the temperature for the fermentation production of succinate by the succinate-producing yeast strain is for example about 25-37°C, for example about 25°C, about 26°C, about 27°C, about 28°C, about 29°C, about 30°C, about 31°C, about 32°C, about 33°C, about 34°C, about 35°C, about 36°C or about 37°C. In one embodiment, the succinate-producing yeast strain of the present invention are fermented at 30°C to produce succinate.

The succinate-producing yeast strain of the present invention may be fermented at a suitable pH known in the art, for example a pH value of less than about 7.0, less than about 6.5, less than about 6.0, less than about 5.5, less than about 5.0, less than about 4.5, less than about 4.0, less than about 3.5, less than about 3.0, less than about 2.5, less than about 2.0, less than about 1.5, less than about 1.0 (for example a pH value of about 1.0-7.0, 1.0-6.0, 1.0-5.5, 1.0-5.0, 1.0-4.5, 1.0-4.0, 1.0-3.5, 1.0-3.0, 2.0-7.0, 2.0-6.0, 2.0-5.5, 2.0-5.0, 2.0-4.5, 2.0-4.0, 2.0-3.5, 2.0-3.0, 3.0-7.0, 3.0-6.0, 3.0-5.5, 3.0-5.0, 3.0-4.5, 3.0-4.0, 3.0-3.5, 4.0-7.0, 4.0-6.0, 4.0-5.5, 4.0-5.0, 4.0-4.5). In one embodiment, the succinate-producing yeast strain of the present invention are fermented at the pH < about 3.0 to produce succinate.

In order to produce succinate, the succinate-producing yeast strain of the present invention can be fermented for a suitable time, for example about 12-96 hours, such as about 12 hours, about 24 hours, about 36 hours, about 48 hours, about 60 hours, about 72 hours, or about 96 hours. In one embodiment, in order to produce succinate, the succinate-producing yeast strain of the present invention may be fermented for about 24-72 hours, for example about 30 hours.

The succinate-producing yeast strain of the present invention may be fermented under the condition of shaking (for example about 100-300 rpm, such as about 150, about 200, or about 250 rpm) to produce succinate.

The content of succinate in a fermentation broth can be determined by any known methods in the art, for example high performance liquid chromatography (HPLC).

In one embodiment, the present invention provides a method for producing succinate, comprising culturing the genetically modified succinate-producing yeast strain in an inorganic salt culture medium (such as containing about 5% w/v glucose and about 30 g/L CaCO₃, or containing about 12% w/v glucose) under the condition of acidic pH (less than about 7.0, less than about 6.5, less than about 6.0, less than about 5.5, less than about 5.0, less than about 4.5, less than about 4.0, less than about 3.5, less than about 3.0, less than about 2.5, less than about 2.0, less than about 1.5, less than about 1.0, for example pH of about 1.0-7.0, 1.0-6.0, 1.0-5.5, 1.0-5.0, 1.0-4.5, 1.0-4.0, 1.0-3.5, 1.0-3.0, 2.0-7.0, 2.0-6.0, 2.0-5.5, 2.0-5.0, 2.0-4.5, 2.0-4.0, 2.0-3.5, 2.0-3.0, 3.0-7.0, 3.0-6.0, 3.0-5.5, 3.0-5.0, 3.0-4.5, 3.0-4.0, 3.0-3.5, 4.0-7.0, 4.0-6.0, 4.0-5.5, 4.0-5.0, 4.0-4.5), optionally comprising isolating and purifying the produced succinate.

In one embodiment, the addition of a neutralizing agent is not requied in the method for producing succinate of the present invention.

In one aspect, the present invention provides use of the genetically modified succinate-producing yeast strain of the present invention or the genetically modified succinate-producing yeast strain prepared by the method for constructing a genetically modified succinate-producing yeast strain according to the present invention in the production of succinate, especially in the production of succinate under the condition of acidic pH (less than about 7.0, less than about 6.5, less than about 6.0, less than about 5.5, less than about 5.0, less than about 4.5, less than about 4.0, less than about 3.5, less than about 3.0, less than about 2.5, less than about 2.0, less than about 1.5, less than about 1.0, for example pH of about 1.0-7.0, 1.0-6.0, 1.0-5.5, 1.0-5.0, 1.0-4.5, 1.0-4.0, 1.0-3.5, 1.0-3.0, 2.0-7.0, 2.0-6.0, 2.0-5.5, 2.0-5.0, 2.0-4.5, 2.0-4.0, 2.0-3.5, 2.0-3.0, 3.0-7.0, 3.0-6.0, 3.0-5.5, 3.0-5.0, 3.0-4.5, 3.0-4.0, 3.0-3.5, 4.0-7.0, 4.0-6.0, 4.0-5.5, 4.0-5.0, 4.0-4.5) and/or without the addition of a neutralizing agent.

As used herein, "optional" or "optionally" refers to occurrence or non-occurrence of a subsequently described event or situation, and such description includes the occurrence and non-occurrence of the event or situation described therein. For example, the optionally comprised step refers to the presence or absence of this step.

As used herein, the term "about" refers to a numerical range that includes specific values, which can be reasonably considered by those skilled in the art to be similar to the specific values. In some embodiments, the term "about" refers to the range within the standard error using measurement accepted typically in the art. In some embodiments, the term "about" refers to +/-10% of a specific value.

The range disclosed herein should be considered to also specifically disclose all possible sub-ranges and various values within such range. For example, the description of the range from 1 to 6 should be deemed as explicitly disclosing sub-ranges from 1 to 3, from 1 to 4, from 1 to 5, from 2 to 4, from 2 to 6 and from 3 to 6, etc, and single numbers within this range, such as 1, 2, 3, 4, 5 and 6. This applies regardless of the breadth of the range.

The present invention will be further illustrated through the following non-limiting examples. It is well known to those skilled in the art that many modifications can be made to the present invention without departing from the spirit of the present application, and such modifications also fall within the scope of the present invention.

Unless otherwise specified, the following experimental methods are conventional methods, and the experimental materials used can be easily obatined from commercial companies.

### Example 1: over-expression of SbMDH gene in Pichia kudriavzevii CY902 ΔURA3 strain

*ΔURA3* mutant was obtained by knocking out the orotidine 5'-phosphate decarboxylase encoding gene *URA3* (at locus *PK2075* of CY902) through homologous recombination (Xi, Y; Zhan, T.; Xu, H.; Chen, J.; Bi, C.; Fan, F.; Zhang, X., Characterization of JEN family carboxylate transporters from the acid-tolerant yeast *Pichia kudriavzevii* and their applications in succinate production. Microb Biotechnol 2021. 0(0), 1-18. doi:10.1111/1751-7915.13781). Subsequently, a CRISPR/Cas9 plasmid pWSPK-Cas9 (GenBank search number: MW296878.1) suitable for this strain was constructed. This plasmid contained the *URA3* screening marker, which allowed positive transformants to be obtained by auxotrophic screening.

CY902 *ΔURA3* was used as the starting strain to over-express the *Sorghum bicolor-derived* malate dehydrogenase SbMDH (Uniprot database search number: P17606) (SEQ ID NO: 1) at the locus *Pk2365* of CY902 genome, wherein the promoter and terminator used were the promoter of the fructose 1,6-biphosphate aldolase-encoding gene *FBA1* (SEQ ID NO: 17) and the terminator of inositol-3-phosphate synthase gene *INO1* (SEQ ID NO: 18) of CY902 itself, respectively. The *SbMDH* gene was synthesized by Nanjing GenScript Biotech Co., Ltd, and was optimized according to CY902 codon preference. A specific construction method was as follows:

### 1. Construction of donor DNA fragments for homologous recombination

Using CY902 genomic DNA as a template, an upstream homologous arm fragment 1 (fragment 1) of a *Pk2365* gene of CY902 itself was amplified with primers 1_UP_2365_F and 1_UP_2365_R1 (see Table 1); the amplification system and procedure were based on the product specification of TAKARA PrimeSTAR^{®}HS DNA polymerase; a *FBA1* promoter sequence (fragment 2) of CY902 itself was amplified with primers 2_P_{FBA1}_F and 2_P_{FBA1}_R (see Table 1); the *Sorghum bicolor* SbMDH-encoding sequence (fragment 3) of was amplified with primers 3_SbMDH_F and 3_SbMDH_R (see Table 1) using a plasmid containing the *SbMDH* synthetic sequence as a template; using CY902 genomic DNA as a template, an *INO1* gene terminator sequence (fragment 4) of CY902 itself was amplified with primers 4_T_{INO1}_F and 4_T_{INOI}_R (see Table 1); a downstream homologous arm fragment 1 (fragment 5) of the *Pk2365* gene of CY902 itself was amplified with primers 5_DW_2365_F1 and 5_DW_2365_R (see Table 1). Using CY902 genomic DNA as a template, an upstream homologous arm fragment 2 (fragment 6) of the *Pk2365* gene of CY902 itself was amplified with primers 1_UP_2365_F and 1_UP_2365_R2 (see Table 1); a downstream homologous arm fragment 2 (fragment 7) of the *Pk2365* gene of CY902 itself was amplified with primers 5_DW_2365_F2 and 5_DW_2365_R (see Table 1).

### 2. Construction of a plasmid for editing Pk2365 gene locus

Using a pWSPK-Cas9 plasmid (GenBank search number: MW296878.1) as a template, a 9052bp plasmid backbone (abbreviated as pWSPK_backbone, SEQ ID NO: 23) without a sgRNA sequence was amplified with primers pWSPK-F and pWSPK-R (see Table 1); the 5'-end sequence (abbreviated as 2365_sgRNA_1) of sgRNA (GenBank search number: MW296878.1) was amplified with primers sgRNA-1F and 2365_sgRNA-1R (see Table 1), and the 3' terminal of said fragment contained a 20nt protospacer specific to the *Pk2365* gene. The 500 bp 3'-sequence (abbreviated as sgRNA_2, SEQ ID NO: 97) of the sgRNA was amplified with primers sgRNA-2F and sgRNA-2R (see Table 1) using a pWSPK-Cas9 plasmid as a template. The above plasmid backbone pWSPK_backbone and sgRNA fragments 2365_sgRNA_1 and sgRNA_2 were ligated by using a seamless cloning kit (Beyotime Biotechnology Co., Ltd., Shanghai, Product Number: D7010S). The seamless cloning ligated product was transferred into Transl-T1 competent cells (TransGen Biotech Co., Ltd., Beijing, product number: CD501-02), and the obtained positive plasmid is named pWSPK_2365.

### 3. Construction of a strain over-expressing SbMDH gene

The pWSPK_2365 plasmid and fragments 6 and 7 were transferred into a CY902 *ΔURA3* strain by a yeast electrotransformation method (doi:10.1111/1751-7915.13781), and a positive transformant was screened and obtained which is named SA101-1 strain (genotype: CY902 *ΔURA3, ΔPk2365*); the pWSPK_2365 plasmid and fragments 1-5 were transferred into a CY902 *ΔURA3* strain by the yeast electrotransformation method, and a positive transformant was screened and obtained which is named SA101-2 strain (genotype: CY902 *ΔURA3, Pk2365::P_{PkFBA1}-ORF_{SbMDH}-T_{PkINO1}*).

**Table 1. Primers required for over-expression of SbMDH gene and construction of CRISPR/Cas9 plasmid**

| Primer Name | Sequence (5'-3'), protospacer underlined | SEQ ID NO |
|---|---|---|
| 1_UP_2365_F | GGTGTCCACTCATATACTTCATTTTC | 24 |
| 1_UP_2365_R1 | ACAATACACATACAATATGGCATCGCTGAATACTATCGAA TCTTGCCAAC | 25 |
| 1_UP_2365_R2 | CTGAATACTATCGAATCTTGCCAAC | 26 |
| 2_P_{FBA1}_F | CGATGCCATATTGTATGTGTATTGT | 27 |
| 2_P_{FBA1}_R | TGTGTTTGTGTTGGGGGTGGTAG | 28 |
| 3_SbMDH_F | ACTACCACCCCCAACACAAACACAATGGGTTTGTCTACC GCATACTCTC | 29 |
| 3_SbMDH_R | CCCTTGAACAAACATCTTGTTGTAGTTAAACTTCACCTGG CAACATAGT | 30 |
| 4_T_{INO1}_F | CTACAACAAGATGTTTGTTCAAGG | 31 |
| 4_T_{INO1}_R | GAAGATTACCATACCATCCCACC | 32 |
| 5_DW_2365_F1 | GATTGGTGGATGGTATGGTAATCTTCGATGAGCATCTAG AGTTGAAGAGGG | 33 |
| 5_DW_2365_F2 | GTTGGCAAGATTCGATAGTATTCAGGATGAGCATCTAGAG TTGAAGAGGG | 34 |
| 5_DW_2365_R | CCGACTTGTACGAATTTGTTCCTC | 35 |
| pWSPK-F | CTTACCAGAATAATATGCAGTTTGCT | 36 |
| pWSPK-R | GATCTCAACAGCGGTAAGATCCTTG | 37 |
| sgRNA-1F | CAAGGATCTTACCGCTGTTGAGATC | 38 |
| 2365_sgRNA-1R | AACTTGCTATTTCTAGCTCTAAAACCTCTCGGATCATCCT TTGGCTGCGCAAGCCCGGAATCGAA | 39 |
| sgRNA-2F | GTTTTAGAGCTAGAAATAGCAAGTTAAAATAAGG | 40 |
| sgRNA-2R | AGCAAACTGCATATTATTCTGGTAAG | 41 |

### Example 2: Over-expression of EcSthA and AoMDH genes in Pichia kudriavzevii CY902 ΔURA3

CY902 *ΔURA3* was used as the starting strain to over-express *Escherichia* coli-derived soluble pyridine nucleotide transhydrogenase EcSthA (EC 1.6.1.1) (SEQ ID NO: 98) at the locus *Pk2365* of CY902 genome, and the promoter and terminator used were the *FBA1* promoter (SEQ ID NO: 17) and the *INO1* terminator (SEQ ID NO: 18) of CY902 itself, respectively. *Aspergillus oryzae*-derived malate dehydrogenase AoMDH (EC 1.1.1.37; Uniprot database search number: I8U0T6) (SEQ ID NO: 99) was over-expressed at the locus of a succinate semialdehyde dehydrogenase (EC 1.2.1.16) gene *PkUGA2,* and the promoter and terminator used were the promoter (SEQ ID NO: 100) of enolase gene *ENO1* and the terminator (SEQ ID NO: 101) of GPI-cell wall glycoprotein encoding gene *SED1* of CY902 itself, respectively. The *EcSthA* and *AoMDH* genes were synthesized by Nanjing GenScript Biotech Co., Ltd, and optimized by CY902 codon preference. A specific construction method was as follows:

### 1. Construction of donor DNA fragments for homologous recombination

A sequence (fragment 8) encoding *Escherichia coli* soluble pyridine nucleotide transhydrogenase EcSthA was amplified with primers 3_EcSthA_F and 3_EcSthA_R (see Table 2) using a plasmid containing the *EcSthA* synthetic sequence as a template; an upstream homologous arm fragment 1 (fragment 9) of a *PkUGA2* gene of CY902 itself was amplified with primers 1_UP_UGA2_F and 1_UP_UGA2_R1 (see Table 2) using CY902 genomic DNA as a template; an *ENO1* promoter sequence (fragment 10) of CY902 itself was amplified with primers 2_P_{ENO1}_F and 2_P_{ENO1}_R (see Table 2); an *Aspergillus oryzae* AoMDH-coding sequence (fragment 11) was amplified with primers 3_AoMDH_F and 3_AoMDH_R (see Table 2) using a plasmid containing the *AoMDH* synthetic sequence as a template; using CY902 genomic DNA as a template, an *SED1* gene terminator sequence (fragment 12) of CY902 itself was amplified with primers 4_T_{SED1}_F and 4_T_{SED1}_R (see Table 1); a downstream homologous arm fragment 1 (fragment 13) of a *PkUGA2* gene of CY902 itself was amplified with primers 5_DW_UGA2_F1 and 5_DW_UGA2_R (see Table 2). Using CY902 genomic DNA as a template, an upstream homologous arm fragment 2 (fragment 14) of a *PkUGA2* gene of CY902 itself was amplified with primers 1_UP_UGA2_F and 1_UP_UGA2_R2 (see Table 2); a downstream homologous arm fragment 2 (fragment 15) of a *PkUGA2* gene of CY902 itself was amplified with primers 5_DW_UGA2_F2 and 5_DW_UGA2_R (see Table 2).

### 2. Construction of a plasmid for editing PkUGA2 gene locus

The 5' end sequence (abbreviated as UGA2_sgRNA_1) of sgRNA was amplified with primers sgRNA-1F and UGA2_sgRNA-1R (see Table 2) using a pWSPK-Cas9 plasmid as a template, and the 3' terminal of said fragment contained a 20nt protospacer specific to *PkUGA2* gene. The pWSPK_backbone, sgRNA_2 and UGA2_sgRNA_1 were ligated by using a seamless cloning kit to finally obtain a positive cloning plasmid which is named pWSPK_UGA2.

### 3. Construction of a strain over-expressing EcSthA and AoMDH genes

The pWSPK_2365 plasmid and fragments 1, 2, 8, 4 and 5 were transferred into a CY902 *ΔURA3* strain by a yeast electrotransformation method, and a positive transformant was screened and obtained which is named SA101-3 strain (genotype: CY902 *ΔURA3, Pk2365::P_{PkFBA1}-ORF_{EcStA}-T_{PkINO1}*). The pWSPK_UGA2 plasmid and fragments 14 and 15 were transferred into SA101-2 and SA101-3 strains by the yeast electroransformation method, and positive transformants were screened and obtained which are named SA102-1 strain (genotype: SA101-2, *ΔUGA2*) and SA102-2 strain (SA101-3, *ΔUGA2*), respectively. The pWSPK_UGA2 plasmid and fragments 9-13 were transferred into a SA101-3 strain by the yeast electrotransformation method, and a positive transformant was screened and obtained which is named SA102-3 strain (genotype: SA101-3, *PkUGA2::P_{PkENO1}-ORF_{AoMDH}-T_{PkSED1}*).

**Table 2. Primers required for over-expression of EcSthA and AoMDH genes and construction of CRISPR/Cas9 plasmid**

| Primer Name | Sequence (5'-3'), protospacer underlined | SEQ ID NO |
|---|---|---|
| 3_EcSthA_F | ACTACCACCCCCAACACAAACACAATGCCACACTCT TATGATTACGACG | 102 |
| 3_EcSthA_R | CCCTTGAACAAACATCTTGTTGTAGTTAAAATAATCT GTTCAAACCGTTCAATGC | 103 |
| 1_UP_UGA2_F | TGCAGTCACACATACACATTAAGCG | 104 |
| 1_UP_UGA2_R1 | GGTGTCTTTGGCGAAGTGTTGTTGGGTAGCTGTGAG GAACGATTACTAC | 105 |
| 1_UP_UGA2_R2 | GGTAGCTGTGAGGAACGATTACTAC | 106 |
| 2_P_{ENO1}_F | CAACAACACTTCGCCAAAGACAC | 107 |
| 2_P_{ENO1}_R | TGTTTGGTTGGAGGGGGTTATATG | 108 |
| 3_AoMDH_F | CATATAACCCCCTCCAACCAAACAATGGTTAAGGCT GCAGTTTTAGGTG | 109 |
| 3_AoMDH_R | CAAAAGAAAGAGCAGTAACCTAATCCACTTACTTTG GTGGTGGGTTCTT | 110 |
| 4_T_{SED1}_F | GTGGATTAGGTTACTGCTCTTTCTTTTG | 111 |
| 4_T_{SED1}_R | CTTGGTTGGATTTGGTATAGTTGAG | 112 |
| 5_DW_UGA2_F1 | CTCTCAACTATACCAAATCCAACCAAGGCTAGAATCT TCAGATTTGGCGA | 113 |
| 5_DW_UGA2_F2 | GTAGTAATCGTTCCTCACAGCTACCGCTAGAATCTTC AGATTTGGCGA | 114 |
| 5_DW_UGA2_R | CAAGAATATGTGATTCATGATGGTGG | 115 |
| UGA2_sgRNA-1 R | AACTTGCTATTTCTAGCTCTAAAACTTGACTAGGGC ACCGGTTCTTGCGCAAGCCCGGAATCGAA | 116 |

### Example 3: Evaluation of succinate production ability of SA101 and SA102 series strains

### 1. Fermentation with a neutralizing agent

A 30 mL yeast inorganic salt culture medium (5% w/v glucose, 30 g/L CaCOs) was inoculated with a strain of SA101 and SA102 series, and subjected to flask shaking fermentation for 24 h under the condition of 30°C and 250 rpm. The titer of succinate was measured by HPLC, as shown in Table 3 below.

**Table 3. Titer of succinate of SA101 and SA102 series strains via flask shaking fermentation**

| Strain | SA101-1 | SA101-2 | SA101-3 | SA102-1 | SA102-2 | SA102-3 |
|---|---|---|---|---|---|---|
| Succinate, g/L | 0.52 | 0.80 | 0.58 | 0.83 | 0.57 | 0.62 |

### 2. Fermentation without a neutralizing agent

A 30 mL yeast inorganic salt culture medium (5% w/v glucose, 0 g/L CaCOs) was inoculated with a strain of SA101 and SA102 series, and subjected to flask shaking fermentation for 24 h under the condition of 30°C and 250 rpm, and then the titer of succinate was measured by HPLC, as shown in Table 4 below.

**Table 4. Titer of succinate of SA101 and SA102 series strains via flask shaking fermentation**

| Strain | SA101-1 | SA101-2 | SA101-3 | SA102-1 | SA102-2 | SA102-3 |
|---|---|---|---|---|---|---|
| Succinate, g/L | 0.31 | 0.52 | 0.38 | 0.54 | 0.40 | 0.42 |

### Example 4: Over-expression of FRD gene in SA101-2 strain

The SA101-2 was used as the starting strain to over-express respectively fumarate reductases of three sources, including (I) FRD of *Saccharomyces cerevisiae* (ScFRD, Uniprot database search number: P32614) (SEQ ID NO: 3); (II) FRD of *Leishmania mexicana* (LmFRD, SEQ ID NO: 4); (III) FRD of *Trypanosoma brucei* (TbFRD, SEQ ID NO: 5), at the locus of *ADH1* gene. The promoter and terminator for over-expressing *FRD* were respectively the promoter (SEQ ID NO: 21) and the terminator (SEQ ID NO: 22) of alcohol dehydrogenase 1 *ADH1* of CY902 itself. The above three *FRD* gene sequences were synthesized by Nanjing GenScript Biotech Co., Ltd, and optimized according to CY902 codon preference. A specific construction method was as follows:

### 1. Construction of donor DNA fragments for homologous recombination

The *ADH1* gene promoter sequence (upstream homologous arm, fragment 16) of CY902 itself was amplified with primers 1_P_{ADH1}_F and 1_P_{ADH1}_R (see Table 5) using CY902 genomic DNA as a template; a ScFRD-encoding sequence (fragment 17-1) of *Saccharomyces cerevisiae* was amplified with primers 2_ScFRD_F and 2_ScFRD_R (see Table 5) using a plasmid containing the *ScFRD* synthetic sequence as a template; a LmFRD-encoding sequence (fragment 17-2) of *Leishmania mexicana,* where 3'-end glyoxysome-localized peptide was truncated, was amplified with primers 2_LmFRD_F and 2_LmFRD_R (see Table 5) using a plasmid containing the *LmFRD* synthetic sequence as a template; a TbFRD-encoding sequence (fragment 17-3) of *Trypanosoma brucei,* where 3'-end glyoxysome-localized peptide was truncated, was amplified with primers 2_TbFRD_F and 2_TbFRD_R (see Table 5) using a plasmid containing the *TbFRD* synthetic sequence as a template; an *ADH1* gene terminator sequence (downstream homologous arm 1, fragment 18) of CY902 itself was amplified with primers 3_T_{ADH1}_F1 and 3_T_{ADHI}_R (see Table 5) using CY902 genomic DNA as a template. An *ADH1* gene terminator sequence (downstream homologous arm 2, fragment 19) of CY902 itself was amplified with primers 3_T_{ADH1}_F2 and 3_T_{ADH1}_R (see Table 5) using CY902 genomic DNA as a template.

### 2. Construction of a plasmid for editing ADH1 gene locus

The 5'-end sequence (abbreviated as ADH1_sgRNA_1) of sgRNA was amplified with primers sgRNA-1F and ADH1_sgRNA-1R (see Table 5) using a pWSPK-Cas9 plasmid as a template, and the 3' terminal of this fragment contained a 20nt protospacer specific to *Pichia kudriavzevii ADH1* (*PkADH1*) gene. The pWSPK _backbone, sgRNA_2 and *ADH1* _sgRNA_1 were ligated by using a seamless cloning kit to finally obtain a positive cloning plasmid which is named pWSPK_ADH1.

### 3. Construction of a strain over-expressing FRD gene

The pWSPK *ADH1* plasmid and fragments 16 and 19 were transferred into a SA101-2 strain according to a yeast electrotransformation method, and a positive cloning transformant was screened and obtained which is named SA103-1 strain (genotype: SA101-2, *ΔADH1*). The pWSPK_ADH1 plasmid and fragments 16, 17 and 18 were transferred into a SA101-2 strain according to the yeast electrotransformation method, and positive transformants were screened and obtained which are named SA103-2 strain (genotype: SA101-2, *ADH1::ORF_{ScFRD}*); SA103-3 strain (genotype: SA101-2, *ADH1:: ORF_{LmFRD}*); SA103-4 strain (genotype: SA101-2, *ADH1:: ORF_{TbFRD}*), respectively.

**Table 5. Primers required for over-expression of FRD gene and construction of CRISPR/Cas9 plasmid**

| Primer Name | Sequence (5'-3'), protospacer underlined | SEQ ID NO |
|---|---|---|
| 1_P_{ADH1}_F | GCAGCCTCTACGTTGAGAATAATGTTCC | 42 |
| 1_P_{ADH1}_R | TTTTTCTAATAGGGGGAGGGGGGGTT | 43 |
| 2_ScFRD_F | ACCCCCCCTCCCCCTATTAGAAAAAATGTCCTTGTCT CCAGTTGTTGTTA | 44 |
| 2_ScFRD_R | AAAGCGGGGAGGTATACACTCTCTAGACTTACTTTCT ATCGTTAGCAATAGATTCAGC | 45 |
| 2_LmFRD_F | ACCCCCCCTCCCCCTATTAGAAAAAATGGCTGATGGT AAAACTTCTGC | 46 |
| 2_LmFRD_R | AAAGCGGGGAGGTATACACTCTCTAGACTTAGGATG GTGGTTCGGTTTCGTC | 47 |
| 2_TbFRD_F | ACCCCCCCTCCCCCTATTAGAAAAAATGGTTGATGGT AGATCTTCCGCA | 48 |
| 2_TbFRD_R | AAAGCGGGGAGGTATACACTCTCTAGACTTAGGAAC CAGATGGTTCGGTTTC | 49 |
| 3_T_{ADH1}_F1 | GTCTAGAGAGTGTATACCTCCCCGCTT | 50 |
| 3_T_{ADH1}_F2 | ACCCCCCCTCCCCCTATTAGAAAAAGTCTAGAGAGT GTATACCTCCCCGCTT | 51 |
| 3_T_{ADH1}_R | CTCTTGAGACAAGCGGAGAGGAAAG | 52 |
| ADH1_sgRNA-1R | AACTTGCTATTTCTAGCTCTAAAACGCTCTAAGGTC TGCAGTCTTTGCGCAAGCCCGGAATCGAA | 53 |

### Example 5: Knockout of PDC1 and GPD1 genes in SA101-2 strain

### 1. Construction of DNA fragments for knocking out PDC1 and GPD1 genes

Using CY902 genomic DNA as a template, upstream and downstream homologous arm fragments (abbreviated as PDC1_1 and PDC1_2) of *PDC1* gene were amplified with primers PDC1_1F and PDC1_1R, PDC1_2F and PDC1_2R (see Table 6); upstream and downstream homologous arm fragments (abbreviated as GPD1_1 and GPD1_2) of *GPD1* gene were amplified with primers GPD1_1F and GPD1_1R, GPD1_2F and GPD1_2R (see Table 6).

### 2. Construction of CRISPR/Cas9 plasmid for editing PDC1 and GPD1 genes

The 5'-end sequence (abbreviated as PDC1_sgRNA_1, SEQ ID NO: 96) of sgRNA was amplified with primers sgRNA-1F and PDC1_sgRNA-1R (see Table 6), and the 3' terminal of this fragment contained a 20 nt protospacer specific to *PDC1* gene. The plasmid backbone pWSPK_backbone and sgRNA fragments PDC1_sgRNA_1 and sgRNA_2 were ligated by using a seamless cloning kit. The seamless cloning ligation product was transferred into a Trans1-T1 competent cell, and the obtained positive plasmid is named pWSPK_PDC1. The 5'-end sequence (abbreviated as GPD1_sgRNA_1) of sgRNA was amplified with primers sgRNA-1F and GPD1_sgRNA-1R (see Table 6), and the 3' terminal of this fragment contained a 20 nt protospacer specific to *GPD1* gene. the pWSPK_backbone, sgRNA_2 and GPD1_sgRNA_1 were fused by seamless cloning to form a pWSPK_GPD1 plasmid for editing the *GPD1* gene.

**Table 6. Primers required for knockout of PDC1 and GPD1 genes and construction of CRISPR/Cas9 plasmid**

| Primer Name | Sequence (5'-3'), protospacer underlined | SEQ ID NO |
|---|---|---|
| PDC1_1F | CCGTATCTTATTGTATCCTATCCTATTC | 54 |
| PDC1_1R | ATGTTCATTTTACATTCAGATGTCAATTTTTATG TTTTGCTGTGTTTTGTTTTA | 55 |
| PDC1_2F | ACATAAAAATTGACATCTGAATGTAAAATGAAC ATTAAA | 56 |
| PDC1_2R | GTTGAAGTCAACGAAGATGGTGAG | 57 |
| PDC1_sgRNA-1R | AACTTGCTATTTCTAGCTCTAAAACGCATTCA ACTCGTTAGCGTTTGCGCAAGCCCGGAATCG AA | 58 |
| GPD1_1F | CTCTACAAGAGAGGAAACACACTACAG | 59 |
| GPD1_1R | AAAATAAATTTAAAATAAACGATATCAAAATT CA | 60 |
| GPD1_2F | GAATTTTGATATCGTTTATTTTAAATTTATTTT ATAACCTCAAGGAGAACTTTGGCATT | 61 |
| GPD1_2R | GAAATCTATGCAAACCACTCTCTCCTG | 62 |
| GPD1_sgRNA-1R | AACTTGCTATTTCTAGCTCTAAAACGCAGATT CATGAGTAAAGGTTGCGCAAGCCCGGAATCG AA | 63 |

### 3. Construction of a strain with PDC1 and GPD1 genes knocked out

The fragments PDC1_1 and PDC1_2 and plasmid pWSPK *PDC1* were simultaneously transferred into a SA101-2. The positive transformant was identified with primer PDC1_1F/PDC1_2R and is named SA104 strain (genotype: SA101-2, *ΔPDC1*). The pWSPK_GPD1 plasmid and fragments GPD1_1 and GPD1_2 were electrotransformed into a SA104 strain, and then a positive transformant was screened with a SD-URA culture medium and obtained, which is named a SA105 strain (genotype: SA104, *ΔGPD1*).

### Example 6: Over-expression of SpMAE1 gene in SA101-2 strain

The *Schizosaccharomyces pombe-derived SpMAE1* transport protein (Uniprot database search number: P50537) was expressed at *MCH4* gene locus of CY902 genome, and the *SpMAE1* gene was synthesized by Nanjing GenScript Biotech Co., Ltd (SEQ ID NO: 2), and optimized according to CY902 codon preference. The promoter and terminator used were the promoter (SEQ ID NO: 19) of glyceraldehyde-3-phosphate dehydrogenase 3 gene *TDH3* and the terminator (SEQ ID NO: 20) of galactose transport protein encoding gene *GAL2* of CY902 itself, respectively. A specific construction method was as follows:

### 1. Construction of donor DNA fragments for homologous recombination

Using CY902 genomic DNA as a template, an upstream homologous arm fragment 1 (fragment 20) of *MCH4* gene was amplified with primers 1_UP_MCH4_F and 1_UP_MCH4_R1 (see Table 7); a *TDH3* gene promoter sequence (fragment 21) of CY902 itself was amplified with primers 2_P_{TDH3}_F and 2_P_{TDH3}_R (see Table 7); a SpMAE1-encoding sequence (fragment 22) of *Schizosaccharomyces pombe* was amplified with primers 3_SpMAE1_F and 3_SpMAE1_R (see Table 7) using a plasmid containing the *SpMAE1* synthetic sequence as a template; using CY902 genomic DNA as a template, a *GAL2* gene terminator sequence (fragment 23) of CY902 itself was amplified with primers 4_T_{GAL2}_F and 4_T_{GAL2}_R (see Table 7); a downstream homologous arm fragment 1 (fragment 24) of *MCH4* gene was amplified with primers 5_DW_MCH4_F1 and 5_DW_MCH4_R (see Table 7). Using CY902 genomic DNA as a template, an upstream homologous arm fragment 2 (fragment 25) of *MCH4* gene was amplified with primers 1_UP_MCH4_F and 1_UP_MCH4_R2 (see Table 7); a downstream homologous arm fragment 2 (fragment 26) of *MCH4* gene was amplified with primers 5_DW_MCH4_F2 and 5_DW_MCH4_R (see Table 7).

### 2. Construction of a plasmid for editing MCH4 of CY902 itself

The 5'-end sequence (abbreviated as MCH4_sgRNA_1) of sgRNA was amplified with primers sgRNA-1F and MCH4_sgRNA-1R (see Table 7) using a pWSPK-Cas9 plasmid as a template, and the 3' terminal of this fragment contained a 20nt protospacer specific to *Pichia kudriavzevii PkMCH4* gene. The pWSPK _backbone, sgRNA_2 and MCH4_sgRNA_1 were ligated by using a seamless cloning kit to finally obtain a positive cloning plasmid which is named pWSPK_MCH4.

**Table 7. Primers required for over-expression of SpMAE1 gene and construction of CRISPR/Cas9 plasmid**

| Primer Name | Sequence (5'-3'), protospacer underlined | SEQ ID NO |
|---|---|---|
| 1_UP_MCH4_F | AACTTTGTTGTACATTAAGCGAGTG | 64 |
| 1_UP_MCH4_R1 | GAAGTGATGTAGATACGCCAACAGGAGCAGTT GATAATGGAAAGGGA | 65 |
| 1_UP_MCH4_R2 | AGCAGTTGATAATGGAAAGGGATAGG | 66 |
| 2_P_{TDH3}_F | CCTGTTGGCGTATCTACATCACTTC | 67 |
| 2_P_{TDH3}_R | TTTTTGTAATTGTGTTTGTTTGTGTGTTTTG | 68 |
| 3_SpMAE1_F | CAAAACACACAAACAAACACAATTACAAAAA ATGGGTGAATTAAAGGAAATCTTAAAGC | 69 |
| 3_SpMAE1_R | ATTAAGACAATTGACAGAAAGTGATTTAGACG GATTCGTGTTCAGAGGA | 70 |
| 4_T_{GAL2}_F | ATCACTTTCTGTCAATTGTCTTAAT | 71 |
| 4_T_{GAL2}_R | GCGATACCGATTTAGCTAGTATTC | 72 |
| 5_DW_MCH4_F1 | GAATACTAGCTAAATCGGTATCGCTGTGTCATT GTTTGTATGGTTTAG | 73 |
| 5_DW_MCH4_F2 | CTATCCCTTTCCATTATCAACTGCTTGTCATTGT TTGTATGGTTTAGGACT | 74 |
| 5_DW_MCH4_R | GTCAACAAATCTGACATCCAATATC | 75 |
| MCH4_sgRNA-1R | AACTTGCTATTTCTAGCTCTAAAACTGGCTAC TTCTGCGGTAACATGCGCAAGCCCGGAATCG AA | 76 |

### 3. Construction of a strain over-expressing SpMAE1 gene

The pWSPK_MCH4 plasmid and fragments 25 and 26 were transferred into a SA101-2 strain according to the yeast electrotransformation method, and a positive transformant was screened and obtained which is named SA106-1 (genotype: SA101-2, *ΔPkMCH4*); the pWSPK_MCH4 plasmid and fragments 20-24 were electrotransformed into a SA101-2 strain, and a positive transformant was screened and obtained which is named SA106-2 (genotype: SA101-2, *PkMCH4::P_{PkTDH3}-ORF_{SpMAE1}-T_{PkGAL2}*).

### Example 7: Over-expression of PYC gene in SA101-2 strain

The *Aspergillus oryzae-derived PYC* (*AoPYC*) gene (Uniprot database search number: Q2UGL1) (SEQ ID NO: 6) was over-expressed at *JEN2-1* locus, and the *PYC1* (*PkPYC1*) gene of CY902 itself was used as a control (SEQ ID NO: 7). The promoter and terminator used were the *TDH3* promoter (SEQ ID NO: 19) and the *GAL2* terminator (SEQ ID NO: 20) respectively. The *AoPYC* gene was synthesized by Nanjing GenScript Biotech Co., Ltd, and optimized according to CY902 codon preference (SEQ ID NO: 6). A specific construction method was as follows:

### 1. Construction of donor DNA fragments for homologous recombination

Using CY902 genomic DNA as a template, an upstream homologous arm fragment 1 (fragment 27) of *JEN2-1* gene was amplified with primers 1_UP_JEN2-1_F and 1_UP_JEN2-1_R1 (see Table 8); a PkPYC1-encoding sequence (fragment 28-1) of CY902 itself was amplified with primers 3_PkPYC1_F and 3_PkPYC1_R (see Table 8); a *Aspergillus oryzae-derived* AoPYC encoding sequence (fragment 28-2) was amplified with primers 3_AoPYC_F and 3_AoPYC_R (see table 8) using a plasmid containing the *AoPYC* synthetic sequence as a template; a downstream homologous arm fragment 1 (fragment 29) of *JEN2-1* gene was amplified with primers 5_DW_JEN2-1_F1 and 5_DW_JEN2-1_R (see Table 8) using CY902 genomic DNA as a template. Using CY902 genomic DNA as a template, an upstream homologous arm fragment 2 (fragment 30) of *JEN2-1* gene was amplified with primers 1_UP_JEN2-1_F and 1_UP_JEN2-1_R2 (see Table 8); a downstream homologous arm fragment 2 (fragment 31) of a *JEN2-1* gene was amplified with primers 5_DW_JEN2-1_F2 and 5_DW_JEN2-1_R (see Table 8).

**Table 8. Primers required for over-expression of PYC gene and construction of CRISPR/Cas9 plasmid**

| Primer Name | Sequence (5'-3'), protospacer underlined | SEQ ID NO |
|---|---|---|
| 1_UP_JEN2-1_F | GATAGGTTCAAAGCCCTCGTCG | 77 |
| 1_UP_JEN2-1_R1 | GAAGTGATGTAGATACGCCAACAGGGGTT GTGTTTCAAGCACCTGACT | 78 |
| 1_UP_JEN2-1_R2 | GGTTGTGTTTCAAGCACCTGACT | 79 |
| 3_PkPYC1_F | CAAAACACACAAACAAACACAATTACAAA AAATGTCAACTGTGGAAGATCACTCCTC | 80 |
| 3_PkPYC1_R | ATTAAGACAATTGACAGAAAGTGATTTAA GCTGCTGGCGCTTCATCTTTG | 81 |
| 3_AoPYC_F | CAAAACACACAAACAAACACAATTACAAA AAATGGCTGCACCATTCAGACAAC | 82 |
| 3_AoPYC_R | ATTAAGACAATTGACAGAAAGTGATTTAA GCCTTAACAATCTTACAAACC | 83 |
| 5_DW_JEN2-1_F1 | GAATACTAGCTAAATCGGTATCGCCCACAC CCCTGTGGACCTGTTCTAT | 84 |
| 5_DW_JEN2-1_F2 | AGTCAGGTGCTTGAAACACAACCCCCTGT GGACCTGTTCTATGTAAAC | 85 |
| 5_DW_JEN2-1_R | ATTCCTCCCATTTCTACCCCCTG | 86 |
| JEN2-1_sgRNA-1R | AACTTGCTATTTCTAGCTCTAAAACTCAAG TGTTCATTATTGTGATGCGCAAGCCCGGA ATCGAA | 87 |

### 2. Construction of a CRISPR/Cas9 plasmid for editing JEN2-1 gene

The 5'-end sequence (abbreviated as JEN2-1_sgRNA_1) of sgRNA was amplified with primers sgRNA-1F and JEN2-1_sgRNA-1R (see Table 8) using a pWSPK-Cas9 plasmid as a template, and the 3' terminal of this fragment contained a 20 nt protospacer specific to *JEN2-1* gene. The pWSPK_backbone and sgRNA_2 in Example 1 and JEN2-1_sgRNA_1 were ligated by using a seamless cloning kit to finally obtain a positive cloning plasmid which is named pWSPK_JEN2-1.

### 3. Construction of a strain over-expressing PYC gene

The pWSPK_JEN2-1 plasmid and fragments 30 and 31 were electrotransformed into a SA101-2 strain, and a positive transformant was screened and obtained which is named SA107-1 (genotype: SA101-2, *ΔPkJEN2-1*); the pWSPK_JEN2-1 plasmid and fragments 27, 21, 28, 23 and 29 were electrotransformed into a SA101-2 strain, and positive transformants were screened and obtained which are named SA107-2 (genotype: SA101-2, *PkJEN2-1::P_{PkTDH3}-ORF_{PkPYC1}-T_{PkGAL2}*); SA107-3 (genotype: SA101-2, *PkJEN2-1: :P_{PkTDH3}-ORF_{AoPYC}-T_{PkGAL2}*), respectively.

### Example 8: Over-expression of PkFUMl gene in SA101-2 strain

The fumarase gene *PkFUMl* (SEQ ID NO: 8) of CY902, where the 5'-end mitochondrion-localized peptide was truncated, was over-expressed at the *PDC1* gene promoter locus of a SA101-2 strain. The promoter and terminator used were the *TDH3* promoter (SEQ ID NO: 19) and the *GAL2* terminator (SEQ ID NO: 20). A specific construction method was as follows:

### 1. Construction of donor DNA fragments for homologous recombination

Using CY902 genomic DNA as a template, an upstream homologous arm fragment (fragment 32) of a *PDC1* gene promoter of CY902 itself was amplified with primers 1_UP_P_{PDC1}_F and 1_UP_P_{PDC1}_R (see Table 9); the *FUM1* encoding sequence (fragment 33), where 5'-end mitochondrion-localized peptide was truncated, was amplified with primers 3_PkFUM1_F and 3_PkFUM1_R (see Table 9); a downstream homologous arm fragment (fragment 34) of a *PDC1* gene promoter of CY902 itself was amplified with primers 5_DW_P_{PDC1}_F and 5_DW_P_{PDC1}_R (see Table 9).

### 2. Construction of a plasmid for editing PDC1 gene promoter locus

The 5'-end sequence (abbreviated as P_{PDC1}_sgRNA_1) of sgRNA was amplified with primers sgRNA-1F and P_{PDC1}_sgRNA-1R (see Table 9) using a pWSPK-Cas9 plasmid as a template, and the 3' terminal of this fragment contained a 20 nt protospacer specific to a *P_{PDC1}* gene promoter. The pWSPK_backbone, sgRNA_2 and P_{PDC1}_sgRNA_1 were ligated by using a seamless cloning kit to finally obtain a positive cloning plasmid which is named pWSPK_P_{PDC1}.

**Table 9. Primers required for over-expression of PkFUMl gene and construction of CRISPR/Cas9 plasmid**

| Primer Name | Sequence (5'-3'), protospacer underlined | SEQ ID NO |
|---|---|---|
| 1_UP_P_{PDC1}_F | GTATCCTCTATCGTATCGTATCGTAG | 88 |
| 1_UP_P_{PDC1_}R | GAAGTGATGTAGATACGCCAACAGGCCATCGTA TCATTTGTAGCCCAC | 89 |
| 3_PkFUM1_F | CAAAACACACAAACAAACACAATTACAAAAAA TGTTCTCAACTACCTCAATTGCA | 90 |
| 3_PkFUM1_R | ATTAAGACAATTGACAGAAAGTGATCTAATCCT TTGGACCAATCATGTTTTC | 91 |
| 5_DW_P_{PDC1}_F | GAATACTAGCTAAATCGGTATCGCTGACATCTGA ATGTAAAATGAACATTAAA | 92 |
| 5_DW_P_{PDC1}_R | GCAAAGACGCAATATTCTCTCTC | 93 |
| P_{PDC1}_sgRNA-1R | AACTTGCTATTTCTAGCTCTAAAACTGTCCTTT TTATACGCCTTCTGCGCAAGCCCGGAATCGAA | 94 |

### 3. Construction of a strain over-expressing PkFUMl gene

The pWSPK_P_{PDC1} plasmid and fragments 32, 21, 33, 23 and 34 were transferred into a SA101-2 strain according to a yeast electrotransformation method, and a positive transformant was screened and obtained which is named SA108 strain (genotype: SA101-2, *P_{PkPDC1::} P_{PkTDH3}-ORF_{PkFUM1}-T_{PkGAL2}*)*.*

### Example 9: Complementary expression of PkURA3 gene in SA101 series strains

An orotidine 5'-phosphate decarboxylase gene *PkURA3* (SEQ ID NO: 117) was complementary at the dicarboxylate transport protein *PkJEN2-2* gene locus of strains of SA101 series. The promoter and terminator used were the promoter (SEQ ID NO: 118) and the terminator (SEQ ID NO: 119) of *PkURA3* itself. A specific construction method was as follows:

### 1. Construction of donor DNA fragments for homologous recombination

Using CY902 genomic DNA as a template, an upstream homologous arm fragment 1 (fragment 35) of a *PkJEN2-2* gene of CY902 itself was amplified with primers 1_UP_JEN2-2_F and 1_UP_JEN2-2_R1 (see Table 10); a *URA3* promoter, the open reading frame and a terminator sequence (fragment 36) were amplified with primers 2_PkURA3_F and 2_PkURA3_R (see Table 10); a downstream homologous arm fragment 1 (fragment 37) of a *PkJEN2-2* gene of CY902 itself was amplified with primers 3_DW_JEN2-2_F1 and 3_DW_JEN2-2_R (see Table 10). Using CY902 genomic DNA as a template, an upstream homologous arm fragment 2 (fragment 38) of a *PkJEN2-2* gene of CY902 itself was amplified with primers 1_UP_JEN2-2_F and 1_UP_JEN2-2_R2 (see Table 10); a downstream homologous arm fragment 2 (fragment 39) of a *PkJEN2-2* gene of CY902 itself was amplified with primers 3_DW_JEN2-2_F2 and 3_DW_JEN2-2_R (see Table 10).

### 2. Construction of plasmid for editing PkJEN2-2 gene site

The 5'-end sequence (abbreviated as JEN2-2_sgRNA_1) of sgRNA was amplified with primers sgRNA-1F and JEN2-2_sgRNA-1R (see Table 10) using a pWSPK-Cas9 plasmid as a template, and the 3' terminal of this fragment contained a 20 nt protospacer specific to a *PkJEN2-2* gene promoter. The pWSPK_backbone, sgRNA_2 and JEN2-2_sgRNA_1 were ligated by using a seamless cloning kit to finally obtain a positive cloning plasmid which is named pWSPK_JEN2-2.

**Table 10. Primers required for complementary expression of PkURA3 gene and construction of CRISPR/Cas9 plasmid**

| Primer Name | Sequence (5'-3'), protospacer underlined | SEQ ID NO |
|---|---|---|
| 1_UP_JEN2-2_F | CCTTGGACCAACCAATAACCTGATTC | 120 |
| 1_UP_JEN2-2_R1 | CTTCCAATATAACTGACGGACTATACCTTGCTAT CTTTTGGTATTGTGCATAATCA | 121 |
| 1_UP_JEN2-2_R2 | TTGCTATCTTTTGGTATTGTGCATAATCA | 122 |
| 2_PkURA3_F | GGTATAGTCCGTCAGTTATATTGGAAGA | 123 |
| 2_PkURA3_R | GAAGACGTTCATGTATGTTTCTGTTG | 124 |
| 3_DW_JEN2-2_F1 | CAACAGAAACATACATGAACGTCTTCCAGCAAT GTTAACCCCAGAAGCT | 125 |
| 3_DW_JEN2-2_F2 | TATGCACAATACCAAAAGATAGCAACAGCAATG TTAACCCCAGAAGCT | 126 |
| 3_DW_JEN2-2_R | GCAGTTTCTGCAAGATTTCACACAG | 127 |
| JEN2-2_sgRNA-1R | AACTTGCTATTTCTAGCTCTAAAACGGCAATGG ATTCAACAAATGTGCGCAAGCCCGGAATCGAA | 128 |

### 3. Construction of a strain with complementary PkURA3 gene

The pWSPK_JEN2-2 plasmid and fragments 38 and 39 were transferred into SA101-1, SA101-2 and SA101-3 strains according to the yeast electrotransformation method, and positive transformants were screened and obtained, which are named SA109-1 strain (genotype: SA101-1, *ΔPkJEN2-2*), SA109-2 strain (genotype: SA101-2, *ΔPkJEN2-2*) and SA109-3 strain (genotype: SA101-3, *ΔPkJEN2-2*), respectively. The pWSPK_JEN2-2 plasmid and fragments 35-37 were transferred into SA101-1, SA101-2 and SA101-3 strains according to the yeast electrotransformation method, and positive transformants were screened and obtained which are named SA109-4 strain (genotype: SA101-1, *PkJEN2-2::P_{PkURA3}-ORF_{PkURA3}-T_{PkURA3}*), SA109-5 strain (genotype: SA101-2, *PkJEN2-2::PPk_{URA3}-ORF_{PkURA3}-T_{PkURA3}*) and SA109-6 strain (genotype: SA101-3, *PkJEN2-2::P_{PkURA3}-ORF_{PkURA3}-T_{PkURA3}),* respectively.

### Example 10: Complementary expression of PkURA3 gene in SA102-SA108 series strains

With reference to example 9, *PkURA3* was complementary at *PkJEN2-2* gene locus of SA102-SA108 strains to obtain SA110-1 strain (genotype: SA102-1, *PkJEN2-2::P_{PkURA3}-ORF_{PkURA3}-T_{PkURA3}*); SA110-2 strain (genotype: SA102-2, *PkJEN2-2::P_{PkURA3}-ORF_{PkURA3}-T_{PkURA3}*); SA110-3 strain (genotype: SA102-3, *PkJEN2-2::P_{pkuRA3}-ORF_{PkURA3}-T_{PkURA3}*); SA111-1 strain (genotype: SA103-1, *PkJEN2-2::P_{PkURA3}-ORF_{PkURA3}-Tp_{kURA3}*); SA111-2 strain (genotype: SA103-2, *PkJEN2-2::P_{PkURA3}-ORF_{PkURA3}-T_{PkURA3}*); SA111-3 strain (genotype: SA103-3, *PkJEN2-2::P_{pkuRA3}-ORF_{PkURA3}-T_{PkURA3}*)*;* SA111-4 strain (genotype: SA103-4, *PkJEN2-2::P_{PkURA3}-ORF_{PkURA3}-T_{PkURA3}*); SA112 strain (genotype: SA104, *PkJEN2-2::P_{pkuRA3}-ORF_{PkURA3}-T_{PkURA3}*); SA113 strain (genotype: SA105, *PkJEN2-2::P_{PkURA3}-ORF_{PkURA3}-T_{PkURA3}*); SA114-1 strain (genotype: SA106-1, *PkJEN2-2::P_{pkuRA3}-ORF_{PkURA3}-T_{PkURA3}*); SA114-2 strain (genotype: SA106-2, *PkJEN2-2::P_{pkuRA3}-ORF_{PkURA3}-T_{PkURA3}*); SA115-1 strain (genotype: SA107-1, *PkJEN2-2::P_{PkURA3}-ORF_{PkURA3}-T_{PkURA3}*); SA115-2 strain (genotype: SA107-2, *PkJEN2-2::P_{pkuRA3}-ORF_{PkURA3}-T_{PkURA3}*); SA115-3 strain (genotype: SA107-3, *PkJEN2-2::P_{PkURA3}-ORF_{PkURA3}-T_{PkURA3}*); and SA116 strain (genotype: SA108, *PkJEN2-2::P_{PkURA3}-ORF_{PkURA3}-T_{PkURA3}*).

### Example 11: Evaluation of succinate production ability of SA101-SA116 series strains

### 1. Fermentation with a neutralizing agent

A 30 mL yeast inorganic salt culture medium (5% w/v glucose, 30 g/L CaCOs) was inoculated with a strain of SA101-SA116 series and then subjected to flask shaking fermentation for 24 h under the condition of 30°C and 250 rpm. The titer of succinate was measured by HPLC.

**Table 11. Titer of succinate of SA101-SA116 series strains via flask shaking fermentation**

| Strain | SA101-1 | SA101-2 | SA101-3 | SA102-1 | SA102-2 | SA102-3 |
|---|---|---|---|---|---|---|
| Succinate, g/L | 0.52 | 0.80 | 0.58 | 0.83 | 0.57 | 0.62 |

| Strain | SA103-1 | SA103-2 | SA103-3 | SA103-4 | SA 104 | SA105 |
|---|---|---|---|---|---|---|
| Succinate, g/L | 0.97 | 1.02 | 2.84 | 9.93 | 1.13 | 1.25 |

| Strain | SA106-1 | SA106-2 | SA107-1 | SA107-2 | SA107-3 | SA108 |
|---|---|---|---|---|---|---|
| Succinate, g/L | 0.82 | 6.07 | 0.80 | 1.04 | 1.12 | 1.57 |

| Strain | SA109-1 | SA109-2 | SA 109-3 | SA 109-4 | SA109-5 | SA109-6 |
|---|---|---|---|---|---|---|
| Succinate, g/L | 0.53 | 0.78 | 0.57 | 0.54 | 0.83 | 0.60 |

| Strain | SA110-1 | SA110-2 | SA110-3 | SA111-1 | SA111-2 | SA111-3 |
|---|---|---|---|---|---|---|
| Succinate, g/L | 0.88 | 0.59 | 0.68 | 1.08 | 1.17 | 3.12 |

| Strain | SA111-4 | SA112 | SA113 | SA114-1 | SA114-2 | SA115-1 |
|---|---|---|---|---|---|---|
| Succinate, g/L | 10.74 | 1.24 | 1.37 | 0.81 | 6.23 | 0.86 |

| Strain | SA115-2 | SA115-3 | SA116 | | | |
|---|---|---|---|---|---|---|
| Succinate, g/L | 1.11 | 1.21 | 1.73 | | | |

### 2. Fermentation without a neutralizing agent

A 30 mL yeast inorganic salt culture medium (5% w/v glucose, 0 g/L CaCOs) was inoculated with a strain of SA101-SA116 series and then subjected to flask shaking fermentation for 24 h under the condition of 30°C and 250 rpm. The titer of succinate was measured by HPLC.

**Table 12. Titer of succinate of SA101-SA116 series strains via flask shaking fermentation**

| Strain | SA101-1 | SA101-2 | SA101-3 | SA102-1 | SA102-2 | SA102-3 |
|---|---|---|---|---|---|---|
| Succinate, g/L | 0.31 | 0.52 | 0.38 | 0.54 | 0.40 | 0.42 |

| Strain | SA103-1 | SA103-2 | SA103-3 | SA103-4 | SA104 | SA105 |
|---|---|---|---|---|---|---|
| Succinate, g/L | 0.59 | 0.62 | 3.28 | 10.63 | 0.93 | 1.11 |

| Strain | SA106-1 | SA106-2 | SA107-1 | SA107-2 | SA 107-3 | SA108 |
|---|---|---|---|---|---|---|
| Succinate, g/L | 0.52 | 5.31 | 0.51 | 0.77 | 0.86 | 1.21 |

| Strain | SA109-1 | SA109-2 | SA109-3 | SA109-4 | SA109-5 | SA109-6 |
|---|---|---|---|---|---|---|
| Succinate, g/L | 0.29 | 0.50 | 0.39 | 0.33 | 0.55 | 0.40 |

| Strain | SA110-1 | SA110-2 | SA110-3 | SA111-1 | SA111-2 | SA111-3 |
|---|---|---|---|---|---|---|
| Succinate, g/L | 0.53 | 0.42 | 0.45 | 0.63 | 0.66 | 3.73 |

| Strain | SA111-4 | SA112 | SA113 | SA114-1 | SA114-2 | SA115-1 |
|---|---|---|---|---|---|---|
| Succinate, g/L | 11.27 | 1.01 | 1.18 | 0.54 | 5.47 | 0.55 |

| Strain | SA115-2 | SA115-3 | SA116 | | | |
|---|---|---|---|---|---|---|
| Succinate, g/L | 0.81 | 0.93 | 1.42 | | | |

### Example 12: Optimization of succinate producing strain

With reference to example 5, the *PDC1* and *GPD1* genes were knocked out in SA103-4 to obtain SA117 strain (genotype: SA103-4, *Δ PDC1*); SA118 strain (genotype: SA117, *ΔGPD1*).

With reference to example 6, the *SpMAE1* gene was over-expressed in SA118 to obtain SA119-1 strain (genotype: SA118, *ΔPkMCH4*); and SA119-2 strain (genotype: SA118, *PkMCH4:: P_{PkTDH3}-ORF_{SpMAE1}-T_{PkGAL2}*).

With reference to example 7, the *PYC* of two sources were over-expressed in SA119-2 strain to obtain SA120-1 strain (genotype: SA119-2, *ΔPkJEN2-1);* SA120-2 strain (genotype: SA119-2, *PkJEN2-1::P_{PkTDH3}-ORF_{PkPYC1}-T_{PkGAL2}*); and SA120-3 strain (genotype: SA119-2, *PkJEN2-1::P_{PkTDH3}-ORF_{AoPYC}-T_{PkGAL2}*).

With reference to example 8, a fumarase gene *PKFUM1,* where 5'-end mitochondrion-localized peptide was truncated, was over-expressed in SA120-3 to obtain SA121 strain (genotype: SA120-3, *P_{PkPDC1}:: P_{PkTDH3}-ORF_{PkFUM1}-T_{PkGAL2}*).

With reference to example 9, an orotidine 5'-phosphate decarboxylase gene *PkURA3* was complementary into SA121 strain to obtain SA122 strain (genotype: SA121, *PkJEN2-2::P_{PkURA3}-ORF_{PkURA3}-T_{PkURA3}*).

With reference to example 4, a *TbFRD* gene was over-expressed in SA102-3 to obtain SA123 strain (genotype: SA102-3, *ADH1:: ORF_{TbFRD}*).

With reference to example 5, *PDC1* and *GPD1* genes were knocked out in SA123 to obtain SA124 strain (genotype: SA123, *ΔPDC1*); and SA125 strain (genotype: SA124, *ΔGPD1*).

With reference to example 6, a *SpMAE1* gene was over-expressed in SA125 to obtain SA126-1 strain (genotype: SA125, *ΔPkMCH4*); and SA126-2 strain (genotype: SA125, *PkMCH4:: P_{PkTDH3}-ORF_{SpMAE1}-T_{PkGAL2}*).

With reference to example 7, *PYC* of two sources was over-expressed in SA126-2 strain to obtain SA127-1 strain (genotype: SA126-2, *ΔPkJEN2-1*); SA127-2 strain (genotype: SA126-2, *PkJEN2-1::P_{PkTDH3}-ORF_{PkPYC1}-T_{PkGAL2}*); and SA127-3 strain (genotype: SA126-2, *PkJEN2-1::P_{pkTDH3}-ORF_{AoPYC}-T_{PkGAL2}*).

With reference to example 8, a fumarase gene *PKFUM1,* where 5'-end mitochondrion-localized peptide was truncated, was over-expressed in SA127-3 to obtain a SA128 strain (genotype: SA127-3, *P_{PkPDC1}:: P_{PkTDH3}-ORF_{PkFUM1}-T_{PkGAL2}*).

With reference to example 9, an orotidine 5'-phosphate decarboxylase gene *PkURA3* was complementary into SA128 strain to obtain SA129 strain (genotype: SA128, *PkJEN2-2::P_{PkURA3}-ORF_{PkURA3}-T_{PkURA3}).*

### Example 13: Evaluation of succinate production ability of SA122 and SA129 strains

SA122 and SA129 strains were respectively subjected to expanded fermentation in a 5L tank (yeast inorganic salt culture medium, 12% w/v glucose). Without any neutralizing agents, the 36 h titer of succinate respectively reached 96.08 g/L and 46.02 g/L, and the yields thereof were respectively 0.89 g/g and 0.57 g/g.

### Example 14: Over-expression of SbMDH gene in Pichia kudriavzevii CICC32244 ΔURA3 strain

With reference to example 1, a CICC32244 (purchased from China Center of Industrial Culture Collection) Orotidine 5'-phosphate decarboxylase encoding gene *URA3* was knocked out to obtain a CICC32244 *ΔURA3* mutant.

The pWSPK_2365 plasmid and fragments 6 and 7 were transferred into a CICC32244 *ΔURA3* strain according to the yeast electrotransformation method and a positive transformant was screened and obtained which is named SA130-1 strain (genotype: CICC32244 *ΔURA3, ΔPk2365*); the pWSPK_2365 plasmid and fragments 1-5 were transferred into a CICC32244 *ΔURA3* strain according to the yeast electrotransformation method and a positive transformant was screened and obtained which is named SA130-2 strain (genotype: CICC32244 *ΔURA3, Pk2365::P_{PkFBA1}-ORF_{SbMDH}-T_{PkINO1}*).

### Example 15: Complementary expression of PkURA3 gene in SA130 series strains

With reference to example 9, *PkURA3* was complementary at the *PkJEN2-2* gene locus of a strain of SA130 series to obtain SA131-1 strain (genotype: SA130-1, *PkJEN2-2:.P_{PkURA3}-ORF_{PkURA3}-T_{PkURA3}*); and SA131-2 strain (SA130-2, *PkJEN2-2::P_{PkURA3}-ORF_{PkURA3}-T_{PkURA3}*).

### Example 16: Evaluation of succinate production ability of SA130 and SA131 series strains

### 1. Fermentation with a neutralizing agent

A 30 mL yeast inorganic salt culture medium (5% w/v glucose, 30g/L CaCOs) were inoculated with a strain of SA130 and SA131 series and subjected to flask shaking fermentation for 24 h under the condition of 30°C and 250 rpm. The titer of succinate was measured by HPLC.

**Table 13. Titer of succinate of SA130 and SA131 series strains via flask shaking ferementation**

| Strain | SA130-1 | SA130-2 | SA131-1 | SA131-2 |
|---|---|---|---|---|
| Succinate, g/L | 0.37 | 0.52 | 0.39 | 0.56 |

### 2. Fermentation without a neutralizing agent

A 30 mL yeast inorganic salt culture medium (5% w/v glucose, 0 g/L CaCOs) was inoculated with a strain of SA130 and SA131 series and subjected to flask shaking fermentation for 24 h under the condition of 30°C and 250 rpm. The titer of succinate was measured by HPLC.

**Table 14. Titer of succinate of SA130 and SA131 series strains via flask shaking fermentation**

| Strain | SA130-1 | SA130-2 | SA131-1 | SA131-2 |
|---|---|---|---|---|
| Succinate, g/L | 0.37 | 0.53 | 0.40 | 0.55 |

### Example 17: Over-expression of SbMDH gene in Saccharomyces cerevisiae BY4742 strain

The *Sorghum bicolor-derived* malate dehydrogenase SbMDH (SEQ ID NO: 1) was over-expressed at the orotidine 5'-phosphate decarboxylase gene *ScURA3* (SEQ ID NO: 129) locus of *Saccharomyces cerevisiae,* and the strain over-expressing *ScURA3* gene was used as a control. The over-expression of the *SbMDH* gene sequence was the same as above and optimized according to CY902 codon preference. The promoter and terminator used were respectively the promoter (SEQ ID NO: 130) of glyceraldehyde 3-phosphate dehydrogenase encoding gene *ScTDH3* and the terminator (SEQ ID NO: 131) of galactose permease gene *ScGAL2* of BY4742 itself. The promoter and terminator used for over-expression of *ScURA3* gene were respectively the promoter (SEQ ID NO: 132) of orotidine 5'-phosphate decarboxylase gene *ScURA3* and the terminator (SEQ ID NO: 133) of GPI-cell wall glycoprotein encoding gene *ScSED1* of BY4742 itself. A specific construction method was as follows:

### 1. Construction of donor DNA fragments for homologous recombination

Using *Saccharomyces cerevisiae* S288c (purchased from ThermoFisher Scientific) genomic DNA as a template, an *ScURA3* upstream homologous arm and an open reading frame (ORF) sequence (fragment 40) were amplified with primers 1_UP_ScURA3_F and 1_ScURA3_R (see Table 15); *ScSED1* terminator sequence 1 (fragment 41) was amplified with primers 2_T_{ScSED1}_F and 2_T_{ScSED1}_R1 (see Table 15); a *ScTDH3* gene promoter sequence (fragment 42) was amplified with primers 3_P_{ScTDH3}_F and 3_P_{ScTDH3_}R (see Table 15); a *Sorghum bicolor-derived SbMDH* encoding sequence (fragment 43) was amplified with primers 4_SbMDH_F and 4_SbMDH_R (see Table 15) using a plasmid containing the *SbMDH* synthetic sequence as a template; using S288c genomic DNA as a template, a *ScGAL2* gene terminator sequence (fragment 44) was amplified with primers 5_T_{ScGAL2}_F and 5_T_{ScGAL2}_R ; a downstream homologous arm fragment 1 (fragment 45) of the *ScURA3* gene was amplified with primers 6_DW_ScURA3_F1 and 6_DW_ScURA3_R (see Table 15). Using S288c genomic DNA as a template, a *ScSED1* terminator sequence 2 (fragment 46) was amplified with primers 2_T_{ScSED1}_F and 2_T_{ScSED1}_R2 (see Table 15); a downstream homologous arm fragment 2 (fragment 47) of the *ScURA3* gene was amplified with primers 6_DW_ScURA3_F2 and 6_DW_ScURA3_R (see Table 15).

### 2. Construction of a Saccharomyces cerevisiae strain over-expressing SbMDH

Fragments 40, 46 and 47 were mixed together and transferred into a BY4742 strain (purchased from ThermoFisher Scientific) according to the yeast electrotransformation method, and a positive transformant was screened and obtained, which is named SA132-1 strain (genotype: BY4742, *ScURA3:: P_{ScURA3}-ORF_{ScURA3}-T_{ScSED1t});* fragments 40-45 were mixed together and transferred into a BY4742 strain according to the yeast electrotransformation method, and a positive transformant was screened and obtained, which is named SA132-2 strain (genotype: BY4742, *ScURA3:: P_{ScURA3}-ORF_{ScURA3}-T_{ScSED1t}-P_{ScTDH3}-ORF_{SbMDH}-T_{ScGAL2}*).

**Table 15. Primers required for over-expression of SbMDH gene in Saccharomyces cerevisiae**

| Primer Name | Sequence (5'-3'), protospacer underlined | SEQ ID NO |
|---|---|---|
| 1_UP_ScURA3_F | GTATTGAGAAGGGCAACGGTTCATC | 134 |
| 1_ScURA3_R | GCGGATGTGTCAAACACCACCGTTAGTTT TGCTGGCCGCATCTTC | 135 |
| 2_T_{ScSED1}_F | ACGGTGGTGTTTGACACATCC | 136 |
| 2_T_{ScSED1_}R1 | CAAATATGTCTTCTTGACCGTACGTGTTTG TTGTTCGCATCTACTGGC | 137 |
| 2_T_{ScSED1}_R2 | GTTTGTTGTTCGCATCTACTGGC | 138 |
| 3_P_{ScTDH3}_F | ACGTACGGTCAAGAAGACATATTTG | 139 |
| 3_P_{ScTDH3}_R | TTTGTTTGTTTATGTGTGTTTATTCG | 140 |
| 4_SbMDH_F | CGAATAAACACACATAAACAAACAAAATG GGTTTGTCTACCGCATACTCTC | 141 |
| 4_SbMDH_R | GATGGAGCGTCTCACTTCAAACGCATTAA ACTTCACCTGGCAACATAGT | 142 |
| 5_T_{ScGAL2}_F | TGCGTTTGAAGTGAGACGCTCCATC | 143 |
| 5_T_{ScGAL2}_R | GTACGAGAAAAGCTCCGAGACGTTG | 144 |
| 6_DW_ScURA3_F1 | CAACGTCTCGGAGCTTTTCTCGTACGCAT GTATACTAAACTCACAAATTAGAGC | 145 |
| 6_DW_ScURA3_F2 | GCCAGTAGATGCGAACAACAAACGCATGT ATACTAAACTCACAAATTAGAGC | 146 |
| 6_DW_ScURA3_R | TACACCTTATCGGCCCAAGCCTT | 147 |

### Example 18: Evaluation of succinate production ability of SA132 series strains

### 1. Fermentation without a neutralizing agent

A 30 mL yeast inorganic salt culture medium (5% w/v glucose, 0g/L CaCOs) was inoculated with a strain of SA132 series and subjected to flask shaking fermentation for 12 h under the condition of 30°C and 250 rpm. The titer of succinate was measured by HPLC.

**Table 16. Flask shaking feremtnation titer of succinate of SA132 series strains**

| Strain | SA132-1 | SA132-2 |
|---|---|---|
| succinate, g/L | 0.12 | 0.22 |

### Example 19: Over-expression of SbMDH gene in Yarrowia lipolytica Polg strain

Since *Yarrowia lipolytica* has an extremely strong ability of non-homologous end joining, a randon insertion into the *Yarrowia lipolytica* genome was utilized to over-express the *Sorghum bicolor-derived* malate dehydrogenase gene *SbMDH* (SEQ ID NO: 1) and the screening marker β-isopropylmalate dehydrogenase (EC 1.1.1.85) gene *YILEU2* (SEQ ID NO: 148), and a *Yarrowia lipolytica* strain with a random insertion of the over-expressed *YILEU2* gene thereinto was used as a control. The over-expression of the *SbMDH* gene sequence was the same as above and optimized according to CY902 codon preference. The promoter and terminator used were respectively the promoter (SEQ ID NO: 149) of glyceraldehyde 3-phosphate dehydrogenase gene *YIGAPDH* and the terminator (SEQ ID NO: 150) of 3-hydroxy-3-methylglutaryl coenzyme A reductase gene *YlHMG1* of Po1g itself. The promoter and terminator used for over-expression of *YILEU2* gene were respectively the promoter (SEQ ID NO: 151) of β-isopropylmalate dehydrogenase gene *YlLEU2* and the terminator (SEQ ID NO: 152) of *Saccharomyces cerevisiae* cytochrome c subtype 1 gene *ScCYC1* of Polg itself. A specific construction method was as follows:

### 1. Construction of donor DNA fragments for homologous recombination

A *YIGAPDH* gene promoter sequence (fragment 48) was amplified with primers 1_P_{YlGAPDH}_F and 1_P_{YlGAPDH}_R (see Table 17) using *Yarrowia lipolytica* W29 genomic DNA (GenBank: GCA_001761485.1) as a template; a *Sorghum bicolor-derived SbMDH* encoding sequence (fragment 49) was amplified with primers 2_SbMDH_F and 2_SbMDH_R (see Table 17) using a plasmid containing the *SbMDH* synthetic sequence as a template; using *Yarrowia lipolytica* W29 genomic DNA as a template, a *YlHMG1* gene terminal sequence (fragment 50) was amplified with primers 3_T_{YlHMG1_}F and 3_T_{YlHMG1_}R (see Table 17); a *YILEU2* promoter and an ORF sequence (fragment 51) were amplified with primers 4_P_{YlLEU2}_F and 4_YlLEU2_R (see Table 17); a *ScCYC1* terminator sequence (fragment 52) was amplified with primers 5_T_{ScCYC1}_F and 5_T_{ScCYC1}_R (see Table 17) using S288c genomic DNA as a template. An expression cassette (fragment 53) over-expressing *SbMDH* and *YILEU2* was amplified with primers 1_P_{YlGAPDH_}F and 5_T_{ScCYC1}_R using fragments 48-52 as templates; an expression cassette (fragment 54) over-expressing *YILEU2* was amplified with primers 4_P_{YlLEU2}_F and 5_T_{ScCYC1}_R using fragments 51 and 52 as templates.

### 2. Construction of a Yarrowia lipolytica strain over-expressing SbMDH

The fragment 54 was transferred into a Po1g strain (purchased from Yeastem Biotechnology Development Co., Ltd) according to the yeast electrotransformation method and a positive transformant was screened and obtained which is named SA133-1 strain (genotype: Polg, *genome:: P_{YlLEU2}-ORF_{YlLEU2}-T_{ScCYC1t}*); fragment 53 was transferred into a Polg strain according to the yeast electrotransformation method and a positive transformant was screened and obtained, which is named SA133-2 strain (genotype: Polg, *genome:: P_{YlGAPDH}-SbMDH-T_{YlHMG1t}-P_{YlLEU2}-ORF_{YlLEU2}-T_{ScCYC1t}*).

**Table 17. Primers required for over-expression of SbMDH gene in Yarrowia lipolytica**

| Primer Name | Sequence (5'-3'), protospacer underlined | SEQ ID NO |
|---|---|---|
| 1_P_{YlGAPDH}_F | AGTAGGATGTCCTGCACGGGTCTTTTTG | 153 |
| 1_P_{YlGAPDH}_R | TGTTGATGTGTGTTTAATTCAAGAATG | 154 |
| 2_SbMDH_F | TCTTGAATTAAACACACATCAACAATGGG TTTGTCTACCGCATACTCTC | 155 |
| 2_SbMDH_R | CTGTTTCCTTCACTATTGTACACCTATTAA ACTTCACCTGGCAACATAGT | 156 |
| 3_T_{YlHMG1}_F | TAGGTGTACAATAGTGAAGGAAACAG | 157 |
| 3_T_{YlHMG1}_R | CTCCACCTGAAATGTTGCTAGAGATG | 158 |
| 4_P_{YlLEU2}_F | TCTCTAGCAACATTTCAGGTGGAGTTCGA CACCATATCATATAAAACTAACA | 159 |
| 4_YlLEU2_R | CTAATTACATGACTCGAGAAGAGATTTATA CACTAGCGGACCCTGCC | 160 |
| 5_T_{ScCYC1}_F | ATCTCTTCTCGAGTCATGTAATTAGTTATG | 161 |
| 5_T_{ScCYC1}_R | GACGCGTCTGTACAGAAAAAAAAG | 162 |

### Example 20: Evaluation of succinate production ability of SA133 series strains

### 1. Fermentation without a neutralizing agent

A 30 mL yeast inorganic salt culture medium (5% w/v glucose, 0 g/L CaCOs) weas inoculated with a stain of SA133 series and subjected to flask shaking fermentation for 24 h under the condition of 30°C and 250 rpm. The titer of succinate was measured by HPLC.

**Table 18. Titer of succinate of SA133 series strains via Flask shaking fermentation**

| Strain | SA133-1 | SA133-2 |
|---|---|---|
| Succinate, g/L | 1.48 | 2.41 |

## Claims

1. A genetically modified succinate-producing yeast strain, having activity or enhanced activity of NADPH-dependent malate dehydrogenase (EC 1.1.1.82),
optionally further having activity or enhanced activity of at least one of: (i) soluble fumarate reductase (EC 1.3.1.6), optionally wherein the 3'-end glyoxysome-localized peptide of said soluble fumarate reductase is partially or completely truncated, (ii) pyruvate carboxylase (EC 6.4.1.1), (iii) fumarase (EC 4.2.1.2), optionally wherein the 5'-end mitochondrion-localized peptide of said fumarase is partially or completely truncated, and (iv) succinate transport protein,
preferably, said NADPH-dependent malate dehydrogenase is derived from a plant, more preferably a C4 plant, more preferably a plant of the family Gramineae, Cyperaceae, Compositae, Euphorbiaceae, Chenopodiaceae, Portulacaceae or Amaranthaceae, or derived from the genus *Euglena* or *Thermobacillus,* more preferably from *Sorghum bicolor, Zea mays, Saccharum officinarum, Pisum sativum, Cicer arietinum, Spinαcia oleracea, Euglena gracilis* or *Methanothermobacter thermautotrophicus,*
more preferably, said NADPH-dependent malate dehydrogenase is a *Sorghum bicolor*-derived NADPH-dependent malate dehydrogenase,
preferably, said soluble fumarate reductase is derived from *Saccharomyces cerevisiae, Trypanosoma brucei, Leishmania mexicana* or *Trypanosoma cruzi,* more preferably *Trypanosoma brucei;*
preferably, said succinate transport protein is selected from a group consisting of *SpMAE1* protein, AnDCT-02 protein, EcDcuB protein and EcDcuC protein, more preferably *SpMAE1* protein;
preferably, said pyruvate carboxylase is derived from *Aspergillus oryzae* or *Pichia kudriαvzevii,* more preferably *Aspergillus oryzae.*

2. The genetically modified succinate-producing yeast strain according to claim 1, further having reduced activity of or inactivated:
(i) pyruvate decarboxylase (EC 4.1.1.43), and/or
(ii) NAD-dependent glycerol 3-phosphate dehydrogenase (EC 1.1.1.8), and/or
(iii) orotidine 5'-phosphate decarboxylase (EC 4.1.1.23), and/or
(iv) monocarboxylate permease, and/or
(v) dicarboxylate transport protein, and/or
(vi) alcohol dehydrogenase 1 (EC 1.1.1.1), and/or
(vii) a bifunctional enzyme of oxaloacetate decarboxylase and 3-hydroxy-3-methylglutarate aldolase (EC 4.1.3.17 or 4.1.1.112).

3. The genetically modified succinate-producing yeast strain according to claim 1 or 2, having:
(i) an over-expressed nucleic acid sequence encoding the NADPH-dependent malate dehydrogenase, preferably said nucleic acid sequence encoding the NADPH-dependent malate dehydrogenase comprising the sequence of SEQ ID NO: 1 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having NADPH-dependent malate dehydrogenase activity, and/or
(ii) an over-expressed nucleic acid sequence encoding the soluble fumarate reductase, preferably said nucleic acid sequence encoding the soluble fumarate reductase comprising a sequence of any one of SEQ ID NOs: 3-5 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having soluble fumarate reductase activity, and/or
(iii) an over-expressed nucleic acid sequence encoding the succinate transport protein, preferably said nucleic acid sequence encoding the succinate transport protein comprising the sequence of SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having succinate transport protein activity, and/or
(iv) an over-expressed nucleic acid sequence encoding the pyruvate carboxylase, preferably said nucleic acid sequence encoding the pyruvate carboxylase comprising the sequence of SEQ ID NO: 6 or 7 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having the pyruvate carboxylase activity, and/or
(v) an over-expressed nucleic acid sequence encoding the fumarase, preferably said nucleic acid sequence encoding the fumarase comprising the sequence of SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having phosphoenolpyruvate carboxylase activity, and/or
(vi) an endogenous gene encoding pyruvate decarboxylase being knocked out, and/or
(vii) an endogenous gene encoding NAD-dependent glycerol 3-phosphate dehydrogenase being knocked out, and/or
(viii) an endogenous gene encoding orotidine 5'-phosphate decarboxylase being knocked out, and/or
(ix) an endogenous gene encoding monocarboxylate permease being knocked out, and/or
(x) an endogenous gene encoding dicarboxylate transport protein being knocked out, and/or
(xi) an endogenous gene encoding alcohol dehydrogenase 1 being knocked out, and/or
(xii) an endogenous gene encoding a bifunctional enzyme of oxaloacetate decarboxylase and 3-hydroxy-3-methylglutarate aldolase being knocked out.

4. The genetically modified succinate-producing yeast strain according to any one of claims 1-3, wherein, in the genetically modified succinate-producing yeast strain,
(a) a nucleic acid sequence encoding the NADPH-dependent malate dehydrogenase and at least one of the following nucleic acid sequences are over-expressed:
a nucleic acid sequence encoding the soluble fumarate reductase;
a nucleic acid sequence encoding the succinate transport protein;
a nucleic acid sequence encoding the pyruvate carboxylase; and
a nucleic acid sequence encoding the fumarase,
(b) a nucleic acid sequence encoding the NADPH-dependent malate dehydrogenase is over-expressed, and an endogenous gene encoding pyruvate decarboxylase and/or an endogenous gene encoding NAD-dependent glycerol 3-phosphate dehydrogenase is knocked out,
(c) a nucleic acid sequence encoding the NADPH-dependent malate dehydrogenase, a nucleic acid sequence encoding the pyruvate carboxylase, a nucleic acid sequence encoding the soluble fumarate reductase and a nucleic acid sequence encoding the succinate transport protein are over-expressed, and an endogenous gene encoding pyruvate decarboxylase and/or an endogenous gene encoding NAD-dependent glycerol 3-phosphate dehydrogenase is knocked out;
(d) a nucleic acid sequence encoding the NADPH-dependent malate dehydrogenase, a nucleic acid sequence encoding the pyruvate carboxylase, a nucleic acid sequence encoding the soluble fumarate reductase, a nucleic acid sequence encoding the fumarase and a nucleic acid sequence encoding the succinate transport protein are over-expressed, and an endogenous gene encoding pyruvate decarboxylase and/or an endogenous gene encoding NAD-dependent glycerol 3-phosphate dehydrogenase is knocked out;
preferably, said nucleic acid sequence encoding the NADPH-dependent malate dehydrogenase is a nucleic acid sequence encoding a *Sorghum bicolor* NADPH-dependent malate dehydrogenase, more preferably comprising the sequence of SEQ ID NO: 1 or a degenerate sequence thereof,
preferably, said nucleic acid sequence encoding the soluble fumarate reductase is a nucleic acid sequence encoding a soluble fumarate reductase derived from *Saccharomyces cerevisiae, Trypanosoma brucei, Leishmania mexicana* or *Trypanosoma cruzi,* more preferably comprising a sequence of any one of SEQ ID NOs: 3-5 or a degenerate sequence thereof,
preferably, said nucleic acid sequence encoding the succinate transport protein is a nucleic acid sequence encoding SpMAE1 protein, more preferably comprising the sequence ofSEQ ID NO: 2 or a degenerate sequence thereof;
preferably, said nucleic acid sequence encoding the pyruvate carboxylase encodes pyruvate carboxylase derived from *Aspergillus oryzae* or *Pichia kudriαvzevii,* more preferably comprising the sequence of SEQ ID NO: 6 or 7 or a degenerate sequence thereof.

5. The genetically modified succinate-producing yeast strain according to any one of claims 1-4, wherein the succinate-producing yeast strain is selected from a group consisting of the genera *Pichia, Rhodotroula, Saccharomyces, Yarrowia, Zygosaccharomyces, Torulopsis* and *Candida,* preferably is selected from a group consisting of the genera *Pichia, Saccharomyces* and *Yarrowia,* more preferably *Pichia kudriavzevii, Saccharomyces cerevisiae* or *Yarrowia lipolytica,* for example the *Pichia kudriavzevii* deposited in China General Microbiological Culture Collection Center (CGMCC) under the deposit number of CGMCC No. 20885.

6. A method for constructing a genetically modified succinate-producing yeast strain, comprising confering activity of or enhancing the activity of an NADPH-dependent malate dehydrogenase (EC 1.1.1.82) to the strain, optionally further comprising confering or enhancing at least one of the following activities: (i) soluble fumarate reductase activity, optionally, the soluble fumarate reductase being freely present in the cytoplasm, (ii) pyruvate carboxylase (EC 6.4.1.1) activity, (iii) fumarase (EC 4.2.1.2) activity, optionally the fumarase being freely present in the cytoplasm, and (iv) succinate transport protein activity,
preferably, said soluble fumarate reductase being derived from *Saccharomyces cerevisiae, Trypanosoma brucei, Leishmania mexicana* or *Trypanosoma cruzi;*
preferably, said succinate transport protein being selected from a group consisting of SpMAE1 protein, AnDCT-02 protein, EcDcuB protein and EcDcuC protein;
preferably, said pyruvate carboxylase being derived from *Aspergillus oryzae* or *Pichia kudriαvzevii,*
preferably, said NADPH-dependent malate dehydrogenase being derived from a plant, more preferably a C4 plant, more preferably a plant of the family Gramineae, Cyperaceae, Compositae, Euphorbiaceae, Chenopodiaceae, Portulacaceae or Amaranthaceae, or being derived from the genus *Euglena* or *Thermobacillus,* and more preferably *Sorghum bicolor, Zea mays, Saccharum officinαrum, Pisum sativum, Cicer αrietinum, Spinαcia oleracea, Euglena gracilis* or *Methanothermobacter thermautotrophicus,*
more preferably, said NADPH-dependent malate dehydrogenase being a *Sorghum bicolor*-deived NADPH-dependent malate dehydrogenase.

7. The method according to claim 6, further comprising attenuating or inactivating in the strain:
(i) pyruvate decarboxylase (EC 4.1.1.43), and/or
(ii) NAD-dependent glycerol 3-phosphate dehydrogenase (EC 1.1.1.8), and/or
(iii) orotidine 5'-phosphate decarboxylase (EC 4.1.1.23), and/or
(iv) monocarboxylate permease, and/or
(v) alcohol dehydrogenase 1 (EC 1.1.1.1), and/or
(vi) dicarboxylate transport protein, and/or
(vii) a bifunctional enzyme of oxaloacetate decarboxylase and 3-hydroxy-3-methylglutarate aldolase (EC 4.1.3.17 or 4.1.1.112).

8. The method according to claim 6 or 7, comprising in the succinate-producing yeast strain:
(i) over-expressing a nucleic acid sequence encoding the NADPH-dependent malate dehydrogenase, preferably said nucleic acid sequence encoding the NADPH-dependent malate dehydrogenase comprising the sequence of SEQ ID NO: 1 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having NADPH-dependent malate dehydrogenase activity, and/or
(ii) over-expressing a nucleic acid sequence encoding the soluble fumarate reductase, preferably, said nucleic acid sequence encoding the soluble fumarate reductase comprising a sequence of any one of SEQ ID NOs: 3-5 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having soluble fumarate reductase activity, and/or
(iii) over-expressing a nucleic acid sequence encoding the succinate transport protein, preferably said nucleic acid sequence encoding the succinate transport protein comprising the sequence of SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having succinate transport protein activity, and/or
(iv) over-expressing a nucleic acid sequence encoding the pyruvate carboxylase, preferably, said nucleic acid sequence encoding the pyruvate carboxylase comprising the sequence of SEQ ID NO: 6 or 7 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity, and/or
(v) over-expressing a nucleic acid sequence encoding the fumarase, preferably, said nucleic acid sequence encoding the fumarase comprising the sequence of SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having phosphoenolpyruvate carboxylase activity, and/or
(vi) knocking out an endogenous gene encoding pyruvate decarboxylase, and/or
(vii) knocking out an endogenous gene encoding NAD-dependent glycerol 3-phosphate dehydrogenase, and/or
(viii) knocking out an endogenous gene encoding orotidine 5'-phosphate decarboxylase, and/or
(ix) knocking out an endogenous gene encoding monocarboxylate permease, and/or
(x) knocking out an endogenous gene encoding dicarboxylate transport protein, and/or
(xi) knocking out an endogenous gene encoding alcohol dehydrogenase 1, and/or
(xii) knocking out an endogenous gene encoding a bifunctional enzyme of oxaloacetate decarboxylase and 3-hydroxy-3-methylglutarate aldolase.

9. The method according to any one of claims 6-8, comprising:
(a) over-expressing a nucleic acid sequence encoding the NADPH-dependent malate dehydrogenase and at least one of the following nucleic acid sequences:
a nucleic acid sequence encoding the soluble fumarate reductase;
a nucleic acid sequence encoding the succinate transport proteins;
a nucleic acid sequence encoding the pyruvate carboxylase; and
a nucleic acid sequence encoding the fumarase,
(b) over-expressing a nucleic acid sequence encoding the NADPH-dependent malate dehydrogenase, and knocking out an endogenous gene encoding pyruvate decarboxylase and/or an endogenous gene encoding NAD-dependent glycerol 3-phosphate dehydrogenase,
(c) over-expressing a nucleic acid sequence encoding the NADPH-dependent malate dehydrogenase, a nucleic acid sequence encoding the pyruvate carboxylase, a nucleic acid sequence encoding the soluble fumarate reductase and a nucleic acid sequence encoding the succinate transport protein, and knocking out an endogenous gene encoding pyruvate decarboxylase and/or an endogenous gene encoding NAD-dependent glycerol 3-phosphate dehydrogenase;
(d) over-expressing a nucleic acid sequence encoding the NADPH-dependent malate dehydrogenase, a nucleic acid sequence encoding the pyruvate carboxylase, a nucleic acid sequence encoding the soluble fumarate reductase, a nucleic acid sequence encoding the fumarase and a nucleic acid sequence encoding the succinate transport protein, and knocking out an endogenous gene encoding pyruvate decarboxylase and/or an endogenous gene encoding NAD-dependent glycerol 3-phosphate dehydrogenase;
preferably, said nucleic acid sequence encoding the NADPH-dependent malate dehydrogenase being a nucleic acid sequence encoding the NADPH-dependent malate dehydrogenase derived from *Sorghum bicolor,* more preferably comprising the sequence of SEQ ID NO: 1 or a degenerate sequence thereof,
preferably, said nucleic acid sequence encoding the soluble fumarate reductase being a nucleic acid sequence encoding the soluble fumarate reductase derived from *Saccharomyces cerevisiae, Trypanosoma brucei, Leishmania mexicana* or *Trypanosoma cruzi,* more preferably comprising a sequence of any one of SEQ ID NOs: 3-5 or a degenerate sequence thereof,
preferably, said nucleic acid sequence encoding the succinate transport protein being a nucleic acid sequence encoding SpMAE1 protein, more preferably comprising the sequence of SEQ ID NO: 2 or a degenerate sequence thereof;
preferably, said nucleic acid sequence encoding the pyruvate carboxylase encodes pyruvate carboxylase derived from *Aspergillus oryzae* or *Pichia kudriαvzevii,* and more preferably comprises the sequence of SEQ ID NO: 6 or 7 or a degenerate sequence thereof,
in the succinate-producing yeast strain.

10. The method according to any one of claims 6-9, wherein the succinate-producing yeast strain is selected from a group consisting of the genera *Pichia, Rhodotroula, Saccharomyces, Yarrowia, Zygosaccharomyces, Torulopsis* and *Candida,* preferably is selected from a group consisting of the genera *Pichia, Saccharomyces* and *Yarrowia,* more preferably *Pichia kudriavzevii, Saccharomyces cerevisiae* or *Yarrowia lipolytica,* for example the *Pichia kudriavzevii* deposited in China General Microbiological Culture Collection Center (CGMCC) under the deposit number of CGMCC No. 20885.

11. A method for producing succinate, comprising culturing the genetically modified succinate-producing yeast strain according to any one of claims 1-5 or the genetically modified succinate-producing yeast strain prepared by the method according to any one of claims 6-10, preferably at pH<3.5, more preferably in a range of pH 1.5-3.5 and/or with no or less addition of a neutralizing agent, optionally isolating and purifying the produced succinate.

12. Use of the genetically modified succinate-producing yeast strain according to any one of claims 1-5 or the genetically modified succinate-producing yeast strain prepared by the method according to any one of claims 6-10 in the production of succinate, preferably in the production of succinate at pH<3.5, more preferably in a range of pH 1.5-3.5 and/or with no or less addition of a neutralizing agent.
